# EUROPEAN PATENT SPECIFICATION

(11) **EP 0 400 440 B1**
(45) Date of publication and mention of the grant of the patent: **13.03.2002**
(21) Application number: 90109596.8
(22) Date of filing: 21.05.1990
(51) Int. Cl.: C07D 307/54, C07D 333/40, C07D 207/40, C07D 213/60, A61K 31/16, A61K 31/38, A61K 31/34, A61K 38/00, A61P 25/08

(54) **Amino acid derivative anticonvulsant**
Anticonvulsive Aminosäure-Derivate
Dérivés anticonvulsifs d'aminoacides

(30) Priority: 19.05.1989 US 354057
(43) Date of publication of application: 05.12.1990
(73) Proprietor: RESEARCH CORPORATION TECHNOLOGIES, INC., Tucson Arizona 85711 (US)
(72) Inventor: Kohn, Harold L., Houston, Texas (US); Watson, Darrell, Flint, Michigan 48504 (US)
(74) Representative: HOFFMANN - EITLE

(56) References cited:
- EP-A- 0 194 464
- EP-A- 0 263 506
- US-A- 3 887 543
- US-A- 4 873 241
- CHEMICAL ABSTRACTS, vol. 96, no. 5, February 1, 1982 Columbus, Ohio, USA VAN DORSSER, WILLIAM et al. "Glycinamides." page 714, column 2, abstract -no. 35 710r
- CHEMICAL ABSTRACTS, vol. 92, no. 7, February 18, 1980 Columbus, Ohio, USA GOLOVENKO, N. YA. et al. "Search for anticonvulsives among compounds metabolized to 1,4-benzodiazepines in the body of animals." page 21, column 1, abstract -no. 51 712r
- CHEMICAL ABSTRACTS, vol. 91, no. 21, November 19, 1979 Columbus, Ohio, USA CZUBA, WLADYSLAW et al. "Hydrolysis of derivatives of (2-pyridyl)acetonitrile." page 644, column 2, abstract -no. 175 147j
- CHEMICAL ABSTRACTS, vol. 101, no. 9, August 27, 1984 Columbus, Ohio, USA ALEKSASHIN, YU. V. et al. "Spectral characteristics of ureides, thioureides, iso- selenoureides, and guani- dines." page 593, column 2, abstract -no. 72 124v

## Description

The present invention relates to compounds and pharmaceutical compositions having central nervous system (CNS) activity which are useful in the treatment of epilepsy and other CNS disorders. More specifically, the compounds of this invention can be characterized as protected amino acid derivatives having the following general formula: 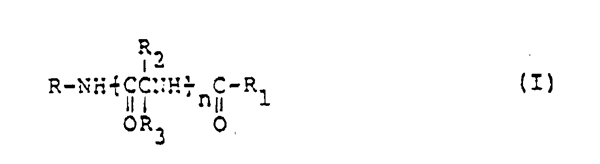 wherein
R is aryl, aryl lower alkyl, heterocyclic or heterocyclic lower alkyl and R is unsubstituted or is substituted with at least one electron withdrawing group, or electron donating group;
R₁ is hydrogen or lower alkyl, unsubstituted or substituted with an electron donating group or an electron withdrawing group and
R₂ and R₃ are independently hydrogen or Z-Y which may be unsubstituted or substituted with at least one electron withdrawing group or one electron donating group, with the proviso that R₂ and R₃ cannot both be hydrogen;
Z is O, S, NR₄ or PR₄;
Y is aryl lower alkyl, lower alkenyl or lower alkynyl, and Y may be unsubstituted or substituted with an electron donating group or an electron withdrawing group; or
ZY taken together is NR₄NR₅R₆, NR₄OR₅, OPR₄R₅, PR₄OR₅, SNR₄R₅, NR₄R₅, NR₄SR₅, SPR₄R₅, PR₄SR₅, NR₄PR₅R₆ or PR₄NR₅R₆;
R₄, R₅ and R₆ are independently hydrogen, lower alkyl, aryl, aryl lower alkyl, lower alkenyl, or lower alkynyl, wherein R₄, R₅ and R₆ may be unsubstituted or substituted with an electron withdrawing group or an electron donating group; and n is 1-4; or wherein
   R is benzyl
   R₁ is methyl
   R₂ is hydrogen
   R₃ is 2-(5-methylfuryl), 2-benzofuryl, 2-benzo[b]-thienyl, 2(5-methylpyrrolyl), or 2-pyridyl; and
   n is 1; or
   wherein
   R is 2-fluorobenzyl, 3-fluorobenzyl, 4-fluorobenzyl, 2,5-difluorobenzyl, or 2,6-difluorobenzyl,
   R₁ is methyl,
   R₂ is hydrogen
   R₃ is furyl, and
   n is 1,
and the pharmaceutical acceptable salts thereof.

The predominant application of anticonvulsant drugs, is the control and prevention of seizures associated with epilepsy or related central nervous system disorders. Epilepsy refers to many types of recurrent seizures produced by paroxysmal excessive neuronal discharges in the brain; the two main generalized seizures are petit mal, which is associated with myoclonic jerks, akinetic seizures, transient loss of consciousness, but without convulsion; and grand mal which manifests in a continuous series of seizures and convulsions with loss of consciousness.

The mainstay of treatment for such disorders has been the long-term and consistent administration of anticonvulsant drugs. Most drugs in use are weak acids that, presumably, exert their action on neurons, glial cells or both of the central nervous system. The majority of these compounds are characterized by the presence of at least one amide unit and one or more benzene rings that are present as a phenyl group or part of a cyclic system.

Much attention has been focused upon the development of anticonvulsant drugs and today many such drugs are well known. For example, the hydantoins, such as phenytoin, are useful in the control of generalized seizures and all forms of partial seizures. The oxazolidinediones, such as trimethadione and paramethadione, are used in the treatment of nonconvulsive seizures. Phenacemide, a phenylacetylurea, is one of the most well known anticonvulsants employed today, while much attention has recently been dedicated to the investigation of the diazepines and piperazines.

EP-A-263 506 concerns anticonvulsant compositions containing amino acid derivatives. The compounds described therein represent α (substituted formic amide) acetic amide derivatives having a substituent at the nitrogen and which may further be substituted at the α C atom with a substituent via RC/C bond.

Furthermore U.S. Patent Nos. 4,002,764 and 4,178,378 to Allgeier, et al. disclose esterified diazepine derivatives useful in the treatment of epilepsy and other nervous disorders. U.S. Patent No. 3,887,543 to Nakanishi, et al. describes a thieno [2,3-e][1,4] diazepine compound also having anticonvulsant activity and other depressant activity. U.S. Patent No. 4,209,516 to Heckendorn, et al. relates to triazole derivatives which exhibit anticonvulsant activity and are useful in the treatment of epilepsy and conditions of tension and agitation. U.S. Patent No. 4,372,974 to Fish, et al. discloses a pharmaceutical formulation containing an aliphatic amino acid compound in which the carboxylic acid and primary amine are separated by three or four units. Administration of these compounds in an acid pH range are useful in the treatment of convulsion disorders and also possess anxiolytic and sedative properties.

Unfortunately, despite the many available pharmacotherapeutic agents, a significant percentage of the population with epilepsy or related disorders are poorly managed. Moreover, none of the drugs presently available are capable of achieving total seizure control and most have disturbing side-effects. Clearly, current therapy has failed to "seize control" of these debilitating diseases.

It is therefore one object of the present invention to provide novel compounds exhibiting CNS activity, particularly anticonvulsant activity.

Another object of this invention is to provide pharmaceutical compositions useful in the treatment of epilepsy and other CNS disorders.

A further object of this invention is to provide a method of treating epilepsy and related convulsant disorders.

These and other objects are accomplished herein by providing compounds of the following general formula: 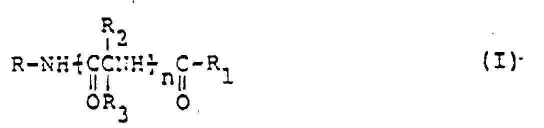 wherein R, R₁, R₂, R₃, R₄, R₅, R₆, Z, Y are as defined hereinabove.

The present invention contemplates employing the compounds of Formula I in compositions of pharmaceutically acceptable dosage forms. Where the appropriate substituents are employed, the present invention also includes pharmaceutically acceptable addition salts. Moreover, the administration of an effective amount of the present compounds, in their pharmaceutically acceptable forms or the addition salts thereof, can provide an excellent regime for the treament of epilepsy, nervous anxiety, psychosis, insomnia and other related central nervous system disorders.

The alkyl groups when used alone or in combination with other groups, exemplary of the substituents are lower alkyl containing from 1 to 6 carbon atoms and may be straight chain or branched. These groups include methyl, ethyl, propyl, isopropyl, butyl, isobutyl, tertiary butyl, amyl and hexyl.

The aryl lower alkyl groups include, for example, benzyl, phenethyl, phenpropyl, phenisopropyl, phenbutyl, and the like, diphenylmethyl, 1,1-diphenylethyl and 1,2-diphenylethyl.

The term aryl refers to an aromatic group which contains up to 18 ring carbon atoms and up to a total of 25 carbon atoms and includes the polynuclear aromatic substituents. These aryl groups may be monocyclic, bicyclic, tricyclic or polycyclic and are fused rings. This group includes phenyl, naphthyl, anthracenyl, phenanthrenyl and azulenyl. It also includes groups like ferrocenyl.

Lower alkenyl is a alkenyl group containing from 2 to 6 carbon atoms and at least one double bond. These groups may be straight chained or branched and may be in the Z or E form. Such groups include vinyl, propenyl, 1-butenyl, isobutenyl, 2-butenyl, 1-pentenyl, (Z)-2-pentenyl, (E)-2-pentenyl, (Z)-4-methyl-2-pentenyl, (E-)-4-methyl-2-pentenyl, pentadienyl, eg., 1,3 or 2,4-pentadienyl.

The term alkynyl include alkyne substituents containing 2 to 6 carbon atoms and may be straight chained as well as branched. It includes such groups as ethynyl, propynyl, 1-butynyl, 2-butynyl, 1-pentynl, 2-pentynyl, 3-methyl-1-pentynl, 3-pentynyl, 1-hexynyl, 2-hexynyl and 3-hexynyl.

The term "electron-withdrawing and electron donating" refer to the ability of a substituent to withdraw or donate electrons relative to that of hydrogen if the hydrogen atom occupied the same position in the molecule. These terms are well understood by one skilled in the art and are discussed in Advanced Organic Chemistry, by J. March, John Wiley and Sons, New York NY, pp. 16-18 (1985). Electron withdrawing groups include halo, including bromo, fluoro, chloro and iodo; nitro, carboxy, lower alkenyl, lower alkynyl, formyl, carboxamido, aryl, quaternary ammonium and the like. Electron donating groups include such groups as hydroxy, lower alkoxy, including methoxy, ethoxy and the like; lower alkyl, such as methyl, ethyl, and the like; amino, lower alkylamino, di(loweralkyl) amino, aryloxy such phenoxy, mercapto, alkylthio, and disulfide. One skilled in the art will appreciate that the aforesaid substituents may have electron donating or electron withdrawing properties under different chemical conditions. Moreover, the present invention contemplates any combination of substituents selected from the above-identified groups.

The term halo includes fluoro, chloro, bromo and iodo.

As employed herein, the heterocyclic substituent contains at least one sulfur, nitrogen or oxygen, but also may include one or several of said atoms. The heterocyclic substituents contemplated by the present invention include heteroaromatics and saturated and partially saturated heterocyclic compounds. These heterocyclics may be monocyclic, bicyclic, tricyclic or polycyclic and are fused rings. The may contain up to 18 ring atoms and up to a total of 17 ring carbon atoms and a total of up to 25 carbon atoms. The heterocyclics are also intended to include the so-called benzoheterocycles. Representative heterocyclics include furyl, thienyl, pyrazolyl, pyrrolyl, imidazolyl, indolyl, thiazolyl, oxazolyl, is othiazolyl, isoxazolyl, piperidyl, pyrrolinyl, piperazinyl, quinolyl, triazolyl, tetrazolyl, isoquinolyl, benzofuryl, benzothienyl, morpholinyl, benzoxazolyl, tetrahydrofuryl, pyranyl, indazolyl, purinyl, indolinyl, pyrazolidinyl, imidazolinyl, imidazolidinyl, pyrrolidinyl, furazanyl, N-methylindolyl, methylfuryl, puridazinyl, pyrimidinyl, pyrazinyl, pyridyl, epoxy, aziridino, oxetanyl, azetidinyl, the N-oxides of the nitrogen containing heterocycles, such as the nitric oxides of pyridyl, pyrazinyl, and pyrimidinyl and the like. The preferred heterocyclic are thienyl, furyl, pyrrolyl, benzofuryl, benzothienyl, indolyl, methylpyrrolyl, morpholinyl. The preferred heterocyclic is a 5 or 6-membered heterocyclic compound. The especially preferred heterocyclic is furyl.

The preferred compounds are those wherein n is 1, but di, tri and tetrapeptides are acceptable.

The preferred values of R is aryl lower alkyl, especially benzyl, and the preferred R₁ is H or lower alkyl. The most preferred R₁ group is methyl.

The most preferred electron donating substituent and electron donating substituent for R₁ e.g., are halo, nitro, alkanoyl, formyl, arylalkanoyl, aryloyl, carboxyl, carbalkoxy, carboxamide, cyano, sulfonyl, sulfoxide, heterocyclic, guanidine, quaternary ammonium, lower alkenyl, lower alkynyl, sulfonium salts, hydroxy, lower alkoxy, lower alkyl, amino, lower alkylamino, di(loweralky)lamino, amino lower alkyl, mercapto and alkylthio.

The ZY groups representative of R₂ and R₃ include alkoxy, such as methoxy, ethoxy, aryloxy, such as phenoxy; thioalkoxy, such as thiomethoxy, thioethoxy; thioaryloxy such as thiophenoxy, alkylamino, such as methylamino, ethylamino, arylamino, such as anilino, lower dialkylamino, such as, dimethylamino, hydrazino, alkylhydrazino and aryl hydrazino, such as N-methylhydrazino and N-phenylhydrazino, and hydroxylamino, such as N-hydroxylamino (-NH-OH) and O-hydroxylamino (-O-NH₂).

It is preferred that at least one of R₂ and R₃ is hydrogen and that the other is heterocyclic. The preferred heterocyclics include furyl, thienyl, benzothienyl, benzofuryl, morpholinyl, indolyl, pyrrolyl, methylpyrrolyl. It is also preferred that one of R₂ and R₃ is methyl, phenyl, isopropyl, 2-thiomethylethyl, ethoxy, methoxy, anilino, propenyl, ethylamino and methylamino.

Preferred compounds of the present invention have the following general formula: 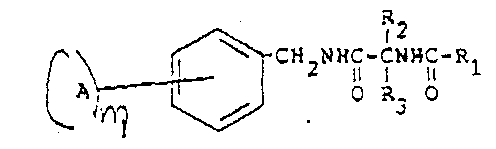 wherein R₁ is H or lower alkyl, R₂ and R₃ are as defined above and A is hydrogen or an electron donating group or electron-withdrawing group and m is 0-5. It is preferred that A is hydrogen (i.e., m=0). However, values of m, equalling 1, 2, or 3 are also preferred.

The compounds of the Formula I have the formula 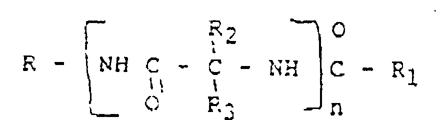 wherein
R is aryl, aryl lower alkyl, heterocyclic or heterocyclic alkyl which is unsubstituted or substituted with at least one electron withdrawing group or at least one electron donating group;
R₁ is hydrogen or lower alkyl which is unsubstituted or substituted with at leat one electron withdrawing group or one electron donating group,
R₂ and R₃ are independently hydrogen or Z-Y which may be unsubstituted or substituted with at least one electron withdrawing group or one electron donating group, with the proviso that R₂ and R₃ cannot both be hydrogen;
Z is 0, S, NR₄ or PR₄;
Y is aryl lower alkyl, lower alkenyl or lower alkynyl, and Y may be unsubstituted or substituted with an electron donating group or an electron withdrawing group; or
ZY taken together is NR₄NR₅R₆, NR₄OR₅, OPR₄R₅, PR₄OR₅, SNR₄R₅, NR₄R₅, NR₄SR₅, SPR₄R₅, PR₄SR₅, NR₄PR₅R₆ or PR₄NR₅R₆;
R₄, R₅ and R₆ are independently hydrogen, lower alkyl, aryl, aryl lower alkyl, lower alkenyl, or lower alkynyl, wherein R₄, R₅ and R₆ may be unsubstituted or substituted with an electron withdrawing group or an electron donating group; and n is 1-4; or wherein
   R is benzyl
   R₁ is methyl
   R₂ is hydrogen
   R₃ is 2-(5-methylfuryl), 2-benzofuryl, 2-benzo[b]-thienyl, 2(5-methylpyrrolyl), or 2-pyridyl; and
   n is 1; or
   wherein
   R is 2-fluorobenzyl, 3-fluorobenzyl, 4-fluorobenzyl, 2,5-difluorobenzyl, or 2,6-difluorobenzyl,
   R₁ is methyl,
   R₂ is hydrogen
   R₃ is furyl, and
   n is 1,
and the pharmaceutical acceptable salts thereof. of this preferred group, it is especially preferred that n is 1.

The compounds of the present invention may contain one (1) or more asymmetric carbons and may exists in racemic and optically active forms. The configuration around each asymmetric carbon can be in either the D or L, form. (It is well known in the art that the configuration around a chiral carbon atoms can also be described as 2 or S in the Cahn-Prelog-Ingold nomenclature system,). All of the various configurations around each asymmetric carbon, including the various enantiomers and diastereomers as well as racemic mixtures and mixtures of enantiomers, diastereomers or both are contemplated by the present invention.

In the principal chain, there exists asymmetry at the carbon atoms to which the groups R₂ and R₃ are attached as substituted. When n is 1, the compounds of the present invention is of the formula 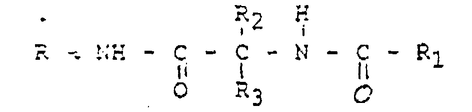 wherein R, R₁, R₂, R₃, R₄, R₅, R₆, Z and Y are as defined previously. As used herein, the term configuration shall refer to the configuration around the carbon atom to which R₂ and R₃ are attached, even though other chiral centers may be present in the molecule. Therefore, when referring to a particular configuration, such as D or L, it is to be understood to mean the stereoisomer, including all possible enantiomers and diastereomers. The compounds of the present invention are directed to the optical isomers, i.e., the compounds of the present invention are either the L-stereoisomer or the D-stereoisomer. These stereoisomers may be found in mixtures of the L and D stereoisomer, e.g., racemic mixtures.

Depending upon the substituents, the present compounds may form addition salts as well. All of these forms are contemplated to be thin the scope of this invention including mixtures of the stereoisomeric forms.

The following three schemes of preparation are generally exemplary of the process which can be employed for the preparation of the present complex: 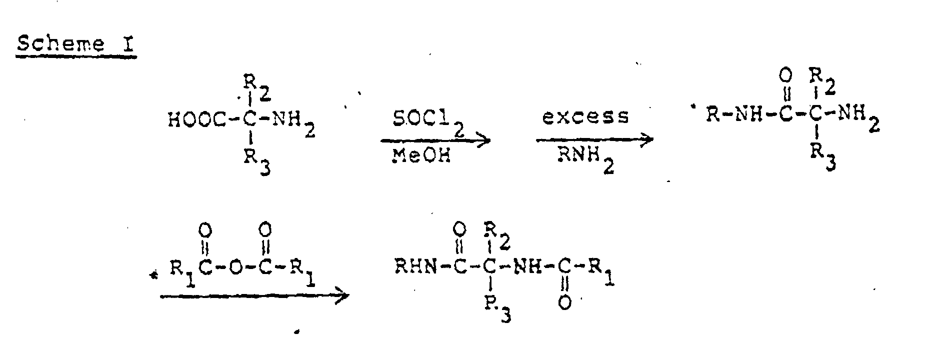 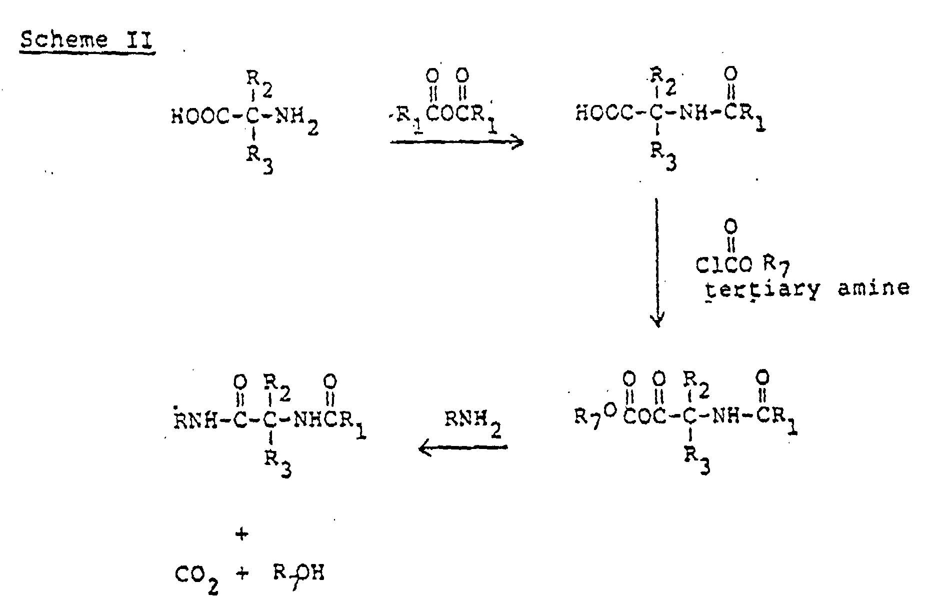 wherein
R₇ = lower alkyl, aryl, aryl lower alkyl, 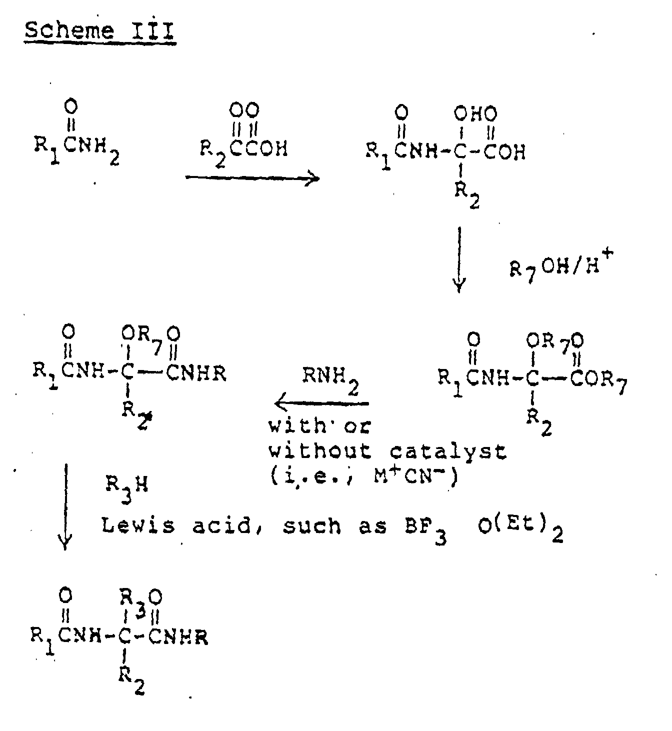 wherein R₃ = aryl, heteroaromatic and R₇ is as defined hereinabove.

More specifically, these compounds can be prepared by art-recognized procedures from known compounds or readily preparable intermediates. For instance, compounds of Formula I can be prepared by reacting amines of Formula II with an acylating derivative of a carboxylic acid of Formula III under amide forming conditions: 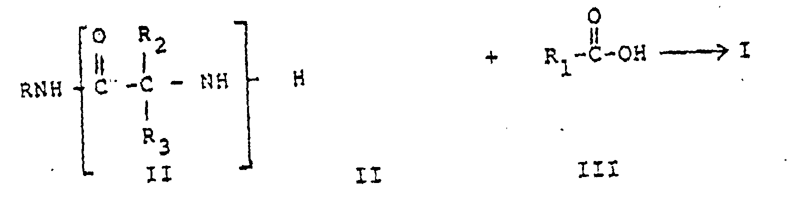 wherein R, R₁, R₂, R₃ and are as defined hereinabove and n = 1.

The amide forming conditions referred to herein involve the use of known derivatives of the described acids, such as the acylhalides, (e.g., 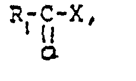 wherein X is Cl, Br, and the like), anhydrides (e.g., 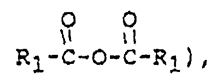 mixed anhydrides, lower alkyl esters, carbodiimides, carbonyldiimidazoles, and the like. It is preferred that the acylating derivative used is the anhydride, 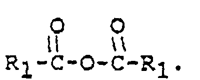 When alkyl esters are employed, amide bond formation can be catalyzed by metal cyanides such as sodium or potassium cyanides.

Another exemplary procedure for preparing compounds wherein at least one of R₂ and R₃ is aromatic or heteroaromatic is as depicted in Scheme IV.

The ester (IV) is reacted with halogen and ultraviolet light in the presence of a catalyst, e.g., AIBN, to form the halo derivative (V). (V) is reacted in the presence, of a Lewis acid, such as zinc chloride, with an aromatic or heteroaromatic compound to form the compound (VI). (VI) in turn is hydrolyzed and then reacted with alkylhaloformace, such as alkylchloroformate in the presence of a tertiary amine to generate the mixed N-acyl amino acid carbonic ester anhydride (VIII). This intermediate is reacted with an amine under amide forming conditions to give the compound of Formula I. Alternatively, (VI) can be reacted directly with an amine (RNH₂) optionally in the presence of a metal catalyst, such as metal cyanides, e.g., potassium or sodium cyanide, under amide forming conditions to form a compound of Formula I. Alternatively, compound VII can be prepared by an independent method and converted to VI which is then reacted with an amine, with or without catalyst to form the compound of Formula I.

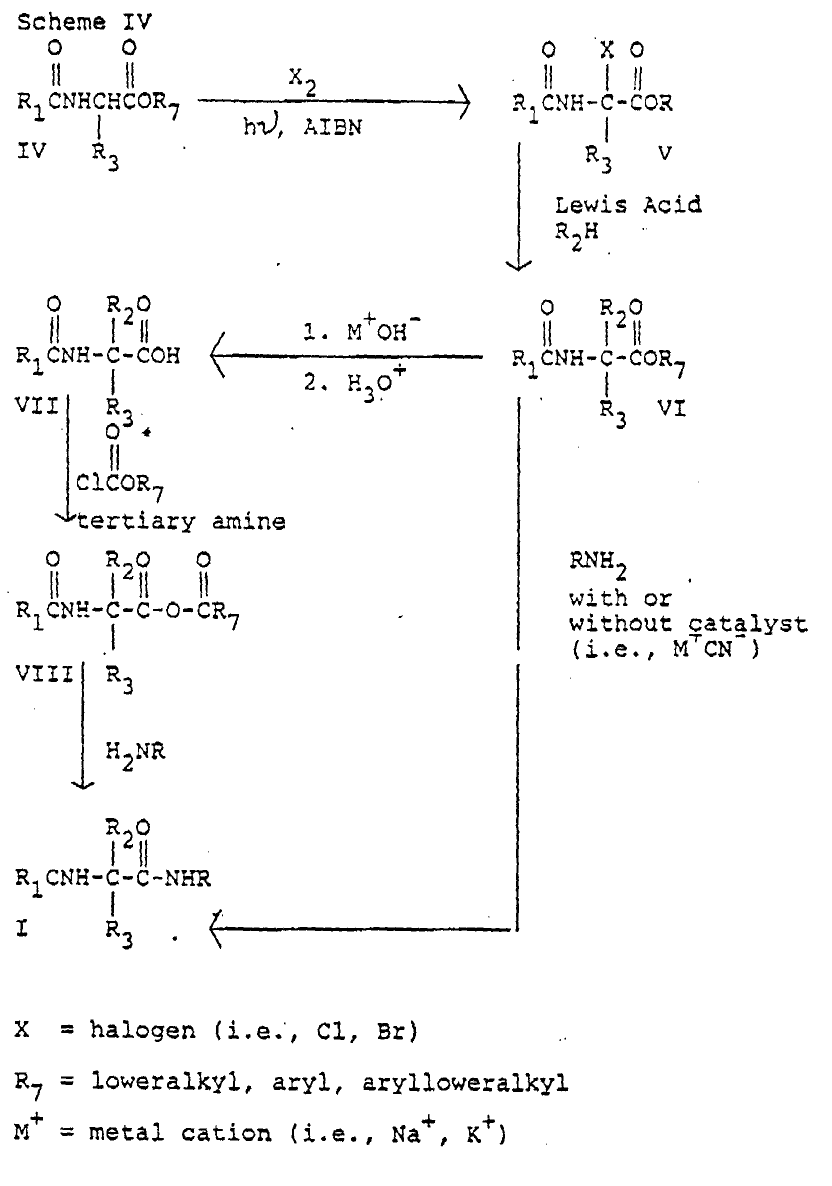

Another useful method for preparing a compound of Formula I involves simple substitution reactions.

An exemplary procedure is as follows: 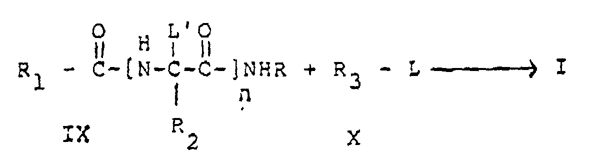 wherein R, R₁, R₂ R₄ and n have the aforesaid meanings and R₃ is defined heretofore except it is not aryl, heteroaromatic or polynuclear aromatic and L and L' are independently a good leaving group, such as halide, tosylates, mesoylates, brosylates and benzyloxy. In this procedure the amine of Formula IX is reacted with a compound of Formula X under substitution conditions. The reaction may take place in the presence of an acid, such as inorganic acid, e.g., hydrochloric acid, sulfuric acid or Lewis acid, such as boron trifluoride and the like or in the presence of a base, such as triethylamine.

However, when R₃ is heteroaromatic, aryl or polynuclear aromatic, L is hydrogen. In the procedure under these circumstances, the reaction should take place in the presence of an acid catalyst, such as an inorganic acid, e.g., hydrochloric acid or a Lewis acid, such as borontrifluoride.

As in any organic reaction, solvents can be employed such as methanol, ethanol, propanol, acetone, tetrahydrofuran, dioxane, dimethylformamide, dichloromethane, chloroform, and the like. The reaction is normally effected at or near room temperature, although temperatures from 0°C up to the reflux temperature of the reaction mixture can be employed.

As a further convenience, the amide forming reaction can be effected in the presence of a base, such as tertiary organic amine, e.g., triethylamine, pyridine, 4-methylmorpholine, picolines and the like, particularly where hydrogen halide is formed by the amide forming reaction, e.g., acyl halide and the amine of Formula II. Of course, in those reactions where hydrogen halide is produced, any of the commonly used hydrogen halide acceptors can also be used.

The exact mineral acid or Lewis acid employed in the reaction will vary depending on the given transformation, the temperature required for the conversion and the sensitivity of the reagent toward the acid in the reaction employed.

As an example of the process described hereinabove, D-(-)-α-acetamido-N-benzyl-2-furanacetamide can be prepared by reacting under amide forming conditions α-acetamide-2-furanacetic acid or an acylating derivative thereof, i.e., esters, e.g. alkyl esters containing 1-6 carbon atoms, or acid anhydrides. The diastereomers formed from the reactions can then be separated by techniques known to one skilled in the art.

The α-acetamido-2-furanacetic acid can be prepared by reacting under substitution conditions, furan with an acylating derivative of 2-acetamido-2-haloacetic acid wherein the halo group is bromo or chloro. By an acylating derivative of 2-acetamido-2-haloacetic acid, it is meant to include lower alkyl esters thereof or the known carboxy protecting groups.

The various substituents on the present new compounds, e.g., as defined in R, R₁, R₂ and R₃ can be present in the starting compounds, added to any one of the intermediates or added after formation of the final products by the known methods of substitution or conversion reactions. For example, the nitro groups can be added to the aromatic ring by nitration and the nitro group converted to other groups, such as amino by reduction, and halo by diazotization of the amino group and replacement of the diazo group. Alkanoyl groups can be substituted onto the aryl groups by Friedel-Crafts acylation. The acyl groups can be then transformed to the corresponding alkyl groups by various methods, including the Wolff-Kishner reduction and Clemmenson reduction. Amino groups can be alkylated to form mono, dialkylamino and trialkylamino groups; and mercapto and hydroxy groups can be alkylated to form corresponding thioethers or ethers, respectively. Primary alcohols can be oxidized by oxidizing agents known in the art to form carboxylic acids or aldehydes, and secondary alcohols can be oxidized to form ketones. Thus, substitution or alteration reactions can be employed to provide a variety of substituents throughout the molecule of the starting material, intermediates, or the final product.

In the above reaction's, if the substituents themselves are reactive, then the substituents can themselves be protected according to the techniques known in the art. A variety of protecting groups known in the art may be employed. Examples of many of these possible groups may be found in "Protective Groups in Organic Synthesis," by T.W. Greens, John Wiley & Sons; 1981.

Resulting mixtures of isomers can be separated into the pure isomers by methods known to one skilled in the art, e.g., by fractional distillation, crystallization and/or chromotagraphy.

The present compounds obviously exist in stereoisomeric forms and the products obtained thus can be mixtures of the isomers, which can be resolved. Optically pure functionalized amino acid derivatives can be prepared directly from the corresponding pure chiral intermediate. Racemic products can likewise be resolved into the optical antipodes, for example, by separation of diastereomeric salts thereof, e.g., by fractional crystallization, by selective enzymatic hydrolysis, e.g., papain digestion, or by use of a chiral stationary phase in chromotagraphy (HPLC). For a discussion of chiral stationary phases for HPLC, See, DeCamp, Chirality, 1, 2-6 (1989), which is incorporated herein by reference with the same force and effect as if fully set forth herein.

For example, a racemic mixture of any of the intermediate in any of the schemes, e.g., 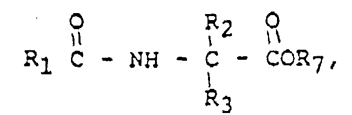 wherein R₇ is H (which can be prepared according to the procedures of Schemes 1, 2, 3, or 4) is reacted with an optically active amine, RNH₂, e.g., (R)(+)-α-methylbenzylamine to form a pair of diastereomeric salts. The diastereomers can then be separated by recognized techniques known in the art, such as chromotagraphy (HPLC) and fractional recrystallization.

In another method, a racemic mixture of final products or intermediates can be resolved by using enzymatic methods. Since enzymes are chiral molecules, it can be used to separate the racemic modification, since it will preferentially act on one of the compounds, without affecting the enantiomer. For example, acylase, such as acylase I, can be used to separate the racemic modification of an intermediate D,L(±)α-acetamido-2-furanacetic acid. It acts on the L (±)α-acetamido-2-furanacetic acid, but will not act on the D enantiomer. In this way, the D(-)α-acetamido-2-furanacetic acid can be isolated. the intermediate can then react with the amine (RNH₂) under amide forming conditions as described hereinabove to form the compound of Formula I.

The active ingredients of the therapeutic compositions and the compounds of the present invention exhibit excellent anticonvulsant activity when administered in amounts ranging from about 10 mg to about 100 mg per kilogram of body weight per day. A preferred dosage regimen for optimum results would be from about 20 mg to about 50 mg per kilogram of body weight per day, and such dosage units are employed that a total of from about 1.0 gram to about 3.0 grams of the active compound for a subject of about 70 kg of body weight are administered in a 24-hour period. This dosage regimen may be adjusted to provide the optimum therapeutic response and is preferably administered one to three times a day in dosages of about 600 mg per administration. For example, several divided doses may be administered daily or the dose may be proportionally reduced as indicated by the exigencies of the therapeutic situation. A decided practical advantage is that the active compound may be administered in an convenient manner such as by the oral, intraveneous (where water soluble), intramuscular or subcutaneous routes.

The active compound may be orally administered, for example, with an inert diluent or with an assimilable edible carrier, or it may be enclosed in hard or soft shell gelatin capsule, or it may be compressed into tablets, or it may be incorporated directly with the food of the diet. For oral therapeutic administration, the active compound may be incorporated with excipients and used in the form of ingestible tablets, buccal tablets, troches, capsules, elixirs, suspensions, syrups, wafers. Such compositions and preparations should contain at least 1% of active compound. The percentage of the compositions and preparations may, of course, be varied and may conveniently be between about 5 to about 80% of the weight of the unit. The amount of active compound in such therapeutically useful compositions is such that a suitable dosage will be obtained. Preferred compositions or preparations according to the present invention are prepared so that an oral dosage unit form contains between about 5 and 1000 mg of active compound.

The tablets, troches, pills, capsules may also contain the following: A binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavoring such *as* cherry or orange flavor. of course, any material used in preparing any dosage unit form should be pharmaceutically pure and substantially non-toxic in the amounts employed. In addition, the active compound may be incorporated into sustained-release preparations and formulations.

The active compound may also be administered parenterally or intraperitoneally. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions (where water soluble) or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid and thimerosal. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

As used herein, "pharmaceutically acceptabie carrier" includes any and all solvents, dispersion media, coatings, antibacterial and antifungal agents, isotonic and absorption delaying agents. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the novel dosage unit forms of the invention arc dictated by and directly dependent on (a) the unique characteristics of the active material and the particular therapeutic effect to be achieved, and (b) the limitations inherent in the art of compounding such an active material for the treatment of disease in living subjects having a diseased condition in which bodily health is impaired as herein disclosed in detail.

The principal active ingredient is compounded for convenient and effective administration in effective amounts with a suitable pharmaceutically acceptable carrier in dosage unit form as hereinbefore disclosed. A unit dosage form can, for example, contain the principal active compound in amounts ranging from 5 to 1000 mg, with from 250 to 750 mg being preferred. Expressed in proportions, the active compound is generally present in from 10 to 750 mg/ml of carrier. In the case of compositions containing supplementary active ingredients, the dosages are determined by reference to the usual dose and manner of administration of the said ingredients.

The compounds of the present invention may be administered in combination with other anti-convulsant agents, such as phenytoin, phenbarbitol, mephenytoin, and phenacemide. This combination is likely to exhibit synergistic effects.

For a better understanding of the present invention together with other and. further objects, reference is made to the following description and examples.

General Methods. Melting points were determined with a Thomas-Hoover melting point apparatus and are uncorrected. Infrared spectra (IR) were run on a Beckman IR-4250 and Perkin-Elmer 1330 and 283 spectrophotometers and calibrated against the 1601-cm⁻¹ band of polysytrene. Absorption values are expressed in waves numbers (cm⁻¹). Proton nuclear magnetic resonance (¹H NMR) spectra were recorded on Varian Associates Models T-60 and FT-80A, General Electric QE 300, and Nicolet NT-300 NMR spectrometers. Carbon nuclear magnetic resonance (¹³C NMR) spectra were run on a Varian Associates Models FT-80A General Electric QW 300 and Nicolet NT-300 instrument. Chemical shifts (δ) are in parts per million (ppm) relative to Me₄Si, and coupling constants (J values) are in hertz. Mass spectral data were obtained at an ionizing voltage of 70 eV on a Hewlett-Packard 5930 gas chromotagraph-mass spectrometer and a Bell-Howell 21-491 spectrometer as well as at the Eli Lilly Laboratories on a Varian MAT-CH-5 spectrometer. High-resolution (EI mode) mass spectra were performed by Drs. James Hudson and John Chinn at the Department of Chemistry, University of Texas at Austin, on a CSC21-1103 double-focusing magnetic-sector spectrometer at 70eV. Elemental analyses were obtained at Spang Microanalytical Laboratories, Eagle Harbor, MI and at the Eli Lilly Research Laboratories.

The solvents and reactants were of the best commercial grade available and were used without further purification unless noted. All anhydrous reactions were run under nitrogen, and all glassware was dried before use. In particular, acetonitrile and triethylamine were distilled from CaH₂, while dichloromethane was distilled from P₂O₅. Acetic anhydride, benzaldehyde and ethyl chloroformate were fractionally distilled.

### Preparation of N-Acetyl-D- and L-amino acid-N-benzylamides.

General Procedure. The D- or L-amino acid amide (11 mmol) was dissolved in dichloromethane (15 mL) and then acetic anhydride (1.23 g, 1.40 mL, 12 mmol) was added dropwise. The solution was stirred at room temperature (18h) and then concentrated to dryness. The residue was recrystallized from chloroform/hexane.

For the following examples, unless otherwise stated, the D-isomer was prepared by the above general procedure followed by separation of the diastereomers, e.g. enzymatically, chromatographic methods and the like, or by one of the procedures outlined on Pages 21-22.

### EXAMPLE 1

### Preparation of D,L-α-Acetamido-N-benzyl-3-thiopheneacetamide.

D,L-α-Acetamido-3-thiopheneacetic acid (2.99 g, 15 mmol) was combined with acetonitrile (60 mL) and the mixture was placed into an ice/salt water bath (-5°C). Triethylamine (1.51 g, 2.10 mL, 15 mmol) was added dropwise, followed by ethyl chloroformate (1.63 g, 1.43 mL, 15 mmol). All additions were done slowly so that the temperature of the mixture did not rise above 0°C. The mixture was then stirred at -5°C (20 min). Benzylamine (1.77 g, 1.80 mL, 16.5 mmol) in acetonitrile (10 mL) was added dropwise and the mixture was stirred at -5°C, (1 h) and then room temperature (18 h).

The mixture was concentrated in vacuo and the residue was combined with hot tetrahydrofuran (50 mL) and cooled in the freezer (3 h), resulting in the formation of a white precipitate. The mixture was filtered and the precipitate was collected, dried in vacuo, and identified as triethylammonium hydrochloride (¹H NMR analysis). The filtrate was concentrated in vacuo and the resulting yellow solid was recrystallized from 1:1 95% ethanol/water.
Yield: 1.91 g (44%). mp 198-199°C.
¹H NMR (DMSO-d₆): δ 1.91 (s, 3H), 4.29 (d,J = 5.2 Hz, 2H), 5.61 (d,J = 7.9 Hz, 1H), 7.15-7.50 (m, 3H), 8.55 (d,J = 7.9 Hz, 1H), 8.74 (t,J = 5.2 Hz, 1H).
¹³C NMR (DMSO-d₆): 22.3, 42.0, 52.5, 122.4, 126.1, 126.7, 127.0 (3C), 128.2 (2C), 139.0, 139.2, 169.0, 169.8 ppm.
IR (KBr): 3460, 1675, 1570, 1400, 720, 695 cm⁻¹.
Mass spectrum, m/e (relative intensity): 288 (2), 245 (3), 155 (88), 112 (100), 91 (31), 85 (17), 65 (7).

| Elemental analysis | | | |
|---|---|---|---|
| Calculated for C₁₅H₁₆N₂O₂S | 62.48% C; | 5.59% H; | 9.71% N. |
| Found | 62.41% C; | 5.47% H; | 9.55% N. |

### EXAMPLE 2

### Preparation of D,L-α-Acetamido-N-benzyl-2-thiopheneacetamide.

N-Acetyl-D,L-ethoxyglycine-N-benzylamide (6.26 g, 25 mmol) was combined with dry ether (175 mL) and then boron trifluoride etherate (5.68 g. 5.0 mL, 40 mmol) was added dropwise, resulting in a homogeneous solution. After stirring a short time, a small amount of a yellow oil separated from the solution. Thiophene (8.41 g, 8.0 mL, 100 mmol) was then added dropwise via syringe and the reaction was stirred at room temperature (4 d). The mixture was cooled in an ice bath and cold aqueous saturated NaHCO₃ (200 mL) was added and the aqueous layer was extracted with ethyl acetate (2 x 100 mL). The organic washings and the original ether layer were combined, dried (Na₂SO₄), and concentrated in vacuo. The residue was purified by flash column chromatography, using 94:6 chloroform/methanol as an eluant (R_{f} = 0.7 94:6 chloroform/methanol), and then recrystallized from benzene.
Yield: 2.67 g (37%).
mp 167-169°C.
¹H NMR (DMSO-d₆): δ 1.91 (s, 3H), 4.31 (d,J = 6.0 Hz, 2H), 5.74 (d,J = 7.9 Hz, 1H), 6.99-7.44 (m, 8H), 8.64 (d,J = 7.9 Hz, 1H), 8.85 (t,J = 6.0 Hz, 1H).
¹³C NMR (DMSO-d₆): 22.4, 42.3, 52.2, 125.6, 125.8, 126.6, 126.9, 127.3 (2C), 128.3 (2C), 139.0, 141.4, 169.2, 169.3 ppm.
Mass spectrum, m/e (relative intensity): 289 (2), 181 (6), 155 (100), 112 (100), 91 (100), 85 (34) , 74 (24).

| Elemental analysis | | | |
|---|---|---|---|
| Calculated for C₁₅H₁₆N₂O₂S | 62.48% C; | 5.59% H; | 9.71% N. |
| found | 62.64% C; | 5.73% H; | 9.61% N. |

### EXAMPLE 3

### Preparation of D,L-α-Acetamido-N-benzyl-2-furanacetamide.

N-Acetyl-D,L-2-(2-furyl)glycine (0.47 g, 2.56 mmol) was combined with acetonitrile (10 mL) and cooled to -5°C (ice/salt water bath). Triethylamine (0.26 g, 0.36 mL, 2.56 mmol) was then rapidly added and the mixture stirred at -5°C (3 min). Ethyl chloroformate (0.28 g, 0.25 mL, 2.56 mmol) was added dropwise beween -4°C and -3°C, and the resulting suspension was stirred at -4°C (20 min), and then an acetonitrile solution (2 mL) of benzylamine (0.30 g, 0.31 mL, 2.82 mmol) was carefully added. During the addition of benzylamine the temperature of the solution did not go above 0°C. The mixture was stirred at -5°C (1 h) and at room temperature (18 h), and then concentrated in vacuo. The residue was then triturated with hot tetrahydrofuran (5 mL), cooled at -16°C (3 h), and the resulting white precipitate was filtered and identified as triethylamine hydrochloride (¹H NMR, 60 MHz, δ 1.00 (t,J = 7.5 Hz, CH₃), 2.82 (q,J = 7.5 Hz, CH₂), 3.83 (s, NH)). The filtrate was evaporated to dryness in vacuo and the resulting oil purified by flash chromatography (98:2 chloroform/methanol) to give 0.09 g (13%) of the desired product as a white solid: R_{f} 0.30 (98:2 chloroform/methanol).
mp 178-179°C.
¹H NMR (300 MHz, DMSO-d₆): δ 1.90 (s, CH₃), 4.31 (d,J = 6.0 Hz, CH₂), 5.58 (d,J = 8.1 Hz, CH), 6.27-6.33 (m, C₃ ^{·}H), 6.40-6.44 (m, C₄ ^{·}H), 7.20-7.36 (m, Ph), 7.60-7.64 (m, C₅ ^{·}H), 8.57 (d,J = 8.1 Hz, NH), 8.73 (t,J = 6.0 Hz, NH).
¹³C NMR (300 MHz, DMSO-d₆): 22.35 (CH₃), 42.27 (CH₂), 50.95 (CH), 107.60 (C₃ ^{·}) , 110 55 (C₄ ^{·}), 126.82 (2C₂ ^{··} or 2C₃ ^{··}), 127.08 (2C₂ ^{··} or 2C₃ ^{··}), 128.27 (C₄ ^{··}), 139.05 (C₁ ^{··}) , 142.58 (C₅ ^{·}),
151.16 (C₂ ^{·}), 168.02 (CH₃CO), 169.30 (NHCO) ppm.
IR (KBr); 3230, 1625 (br), 1525 (br), 1375 (br) 1230, 1090, 890 cm⁻¹.
Mass spectrum, m/e (relative intensity): 273 (1) , 139 (100), 96 (94), 91 (51) 65 (9).

| Elemental analysis | | | |
|---|---|---|---|
| Calculated for C₁₅H₁₆N₂O₃ | 66.16% C; | 5.83% H; | 10.29% N. |
| Found | 65.92% C; | 5.83% H; | 10.15% N. |

### EXAMPLE 4

### Preparation of D,L-α-Acetamido-N-benzyl-2-pyrroleacetamide.

2-Acetamido-N-benzyl-2-ethoxyacetamide (2.00 g, 8.0 mnol) was suspended in anhydrous ethyl ether (60 mL), and then boron trifluoride etherate (1.82 g, 1.57 mL, 12.8 mmol) was added in one portion and the resulting solution was stirred (15 min). The pyrrole (2.14 g, 2.22 mL, 32 mmol) was then added in one portion and the solution was stirred at room temperature (48 h) during which time a precipitate formed. Hexanes (80 mL) were then added to the suspension, and the mixture was filtered and the brown semi-solid was triturated with 95:5 chloroform/methanol (30 mL) to furnish a green solid. This material was purified by flash chromatography (95:5 chloroform/methanol) to yield 0.94 g (35°) of the desired product as a white solid: R_{f} 0.29 (96:4 chloroform/methanol)
mp 174-175°C.
1^{H} NMR (300 MHz, CD₃CN) : δ 1.93 (s, CH₃), 4.35 (d,J = 6.0 Hz, CH₂), 5.42 (d,J = 6.9 Hz, CH), 6.00-6.18 (m, C₃ ^{·}H, C₄ ^{·}H, 6.68-6.72 (m, C₅ ^{·}H) , 7.04 (d,J = 6.9 Hz, NH), 7.17 (t,J = 6.0 Hz, NH), 7.10-7.47 (m, Ph), 9.10-9.80 (br s, NH).
¹³C NMR (300 MHz, CD₃CN): 23.02 (CH₃), 43.83 (CH₂), 52.65 (CH), 107.57 (C₃ ^{·}), 108.85 (C₄ ^{·}), 119.33 (C₅ ^{·}), 127.96 (C₂ ^{·}), 128.01 (2C₂ ^{··}or 2C₃ ^{··}), 128.09 (2C₂ ^{··} or 2C₃ ^{··}), 129.49 (C₄ ^{··}), 140,01 (C₁ ^{··}), 170.94 (COCH₃), 171.21 (CONH) ppm.
IR (KBr) 3320, 1570 (br), 1470 (br), 1330, 1230, 950, 890, 860, 760, 710, 690, 655 cm⁻¹.
Mass spectrum, m/e (relative intensity): 171 (12), 228 (2), 213 (1), 180 (2), 164 (9), 137 (93), 108 (20), 95 (100), 91 (38), 82 (35), 68 (15).

| High resolution mass spectral analysis | |
|---|---|
| Calculated for C₁₅H₁₇N₃O₂ | 271.13208. |
| Found | 271.13144. |

### EXAMPLE 5

### Preparation of D,L-2-Acetamido-N-benzyl-2-ethoxyacetamide.

An ethanolic solution (420 mL) of ethyl 2-acetamido-2-ethoxyacetate (27.92 g, 147 mmol) and benzylamine (23.70 g, 24 mL, 221 mmol) was stirred at 40-45°C for 3 days. The reaction mixture was evaporated in vacuo and the residue recrystallized (1:3.5 tetrahydrofuran/hexanes (650 mL)) to yield 25.80 g (70%) of the desired product as beige crystals: R_{f} 0.59 (95:5 chloroform/methanol).
mp 153-155°C.
¹H NMR (300 MHz, CDCl₃): δ 1.20 (t,J = 7.0 Hz, CH₃), 2.07 (s, CH₃), 3.60-3.76 (m, CH₂CH₃) 4.40-4.54 (m, CH₂NH), 5.60 (d,J = 8.7 Hz, CH), 6.63 (d,J = 8.7 Hz, NH), 7.00 (br s, NH), 7.26-7.36 (m, Ph).
¹³C NMR (300 MHz, CDCl₃): 15.06 (CH₃CH₂), 23.25 (CH₃CO), 43.60 (CH₂NH), 64.51 (CH₂CH₃), 77.43 (CH), 127.69 (2C₂ ^{··} or 2C ^{··}, C₄ ^{··}), 128.79 (2C₂ ^{··}or 2C₃ ^{··}), 137.57 (C₁ ^{··}), 168.13 (COCH₃), 171.29 (CONH) ppm.
IR (KBr): 3260, 1630 (br), 1550 (sh), 1505 (br), 1380, 1360, 1230, 1115, 1060, 1015, 890/ 745, 690 cm⁻¹.
Mass spectrum, m/e (relative intensity): 251 (4), 163 (9), 116 (98) , 106 (34), 91 (98) , 74 (100).

| Elemental analysis | | | |
|---|---|---|---|
| Calculated for C₁₃H₁₈N₂O₃ | 62.38% C; | 7.25% H; | 11.19% N. |
| Found | 62.49% C; | 7.27% H; | 11.24% N. |

### EXAMPLE 6

### Preparation of D,L-2-Acetamido-N-benzyl-2-methoxyacetamide.

To a methanolic solution (180 mL) of methyl 2-acetamido-2-methoxyacetate (8.73 g, 54 mmol) was rapidly added benzylamine (8.68 g, 8.80 mL, 81 mmol) and then the mixture was stirred at 50°C (3 days) during which time a beige precipitate appeared. The solvent was removed in vacuo and the resulting precipitate was recrystallized from tetrahydrofuran (2x) to give 7.67 g (32%) of the desired product as beige crystals: R_{f} 0.35 (95:5 chloroform/methanol).
mp 145-.146°C.
¹H NMR (300 MHz, CDCl₃): δ 2.06 (s, CH₃CO) , 3.37 (s, CH₃O), 4.40-4.35 (m, CH₂), 5.52 (d,J = 8.7 Hz, CH), 7.12 (d,J = 8.7 Hz, NH), 7.20-7.40 (m, Ph, NH).
¹³C NMR (300 MHz, CDCl₃) : 23.03 (CH₃CO), 43.51 (CH₂), 55.84 (CH₃O), 78.94 (CH), 127.62 (C₄ ^{··}), 127.70 (2C₂ ^{··}or 2C₃ ^{··}) , 128.70 (2C₂ ^{··} or 2C₃ ^{··}), 137.45 (C₁ ^{··}) 166.91 (COCH₃), 171.57 (CONH) ppm.
IR (KBr): 1260, 1825 (br), 1550, 1505, 1435, 1390, 1370, 1230, 1120, 1050, 935, 890, 690 cm⁻¹.
Mass spectrum, m/e (relative intensity): 237 (1), 205(2), 177 (2), 163 (4), 146 (1), 134 (1), 121 (2), 106 (26), 102 (98), 91 (95), 77 (13), 61 (100).

| Elemental analysis | | | |
|---|---|---|---|
| Calculated for C₁₂H₁₆N₂O₃ | 61.00% C; | 6.83% H; | 11.86% N. |
| | 60.91% C; | 6.85% H; | 11.66% N. |

### EXAMPLE 7

### Preparation of (D,L)-α-Acetamido-N-benzyl-2-(5-methylfuran)acetamide.

N-Acetyl-D,L-ethoxyglycine-N-benzylamide (2.00 g, 8.0 mmol) was suspended in anhydrous ethyl ether, and then boron trifluoride etherate (1.82 g, 12.8 mmol) was rapidly added, and the resulting solution was stirred for 15 min. The 2-methylfuran (2.63 g, 32.0 mmol) was then added and the reaction was stirred at room temperature (3 d). The reaction mixture was poured into an aqueous saturated NaHCO₃ solution and extracted with ethyl acetate (3 x). The ethyl acetate extracts were combined, dried (Na₂SO₄) and evaporated in vacuo to give a beige solid, which was purified by flash chromatography (98:2 chloroform/methanol) to give the desired product as a white crystalline solid.
Yield: 1.40 g (61%)
R_{f} 0.25 (98:2 chloroform/methanol).
mp 148-150 °C.
¹H NMR (DMSO-d₆) δ 1.88 (s, CH₃CO), 2.23 (s, CH₃), 4.24-4.36 (m, CH₂), 5.49 (d, *J* = 8.0 Hz, CH), 6.01 (br s, C_{3'}-H), 6.14 (d, *J* = 2.4 Hz, C_{4'}H), 7.20-7.31 (m, Ph), 8.52 (d, J =8.0 Hz, NH), 8.69 (t, *J* = 5.6 Hz, NH).
¹³_{C} NMR (DMSO-d₆) 13.44 (CH₃), 22.35 (CH₃CO), 44.11 (CH₂), 53.23 (CH), 107.51 (C_{3'} or C_{4'}), 110.40 (C_{3'} or C_{4'}), 128.13 (C_{4"}), 128.18 (2C_{2"} or 2C_{3"}), 129.43 (2C_{2"} or 2C_{3"}), 139.69 (C_{1"}), 149.18 (C_{2'} or C_{5'}), 153.81 (C_{2'} or C_{5'}), 170.78 (CH₃CO), 173.03 (CONH) ppm.
IR (KBr) 3270, 1620 (br), 1520 (br), 1440, 1360, 1210, 1010 cm⁻¹.
Mass spectrum, m/e (relative intensity) 286 (3), 179(8), 153 (57), 152 (57), 111 (23) 110 (100), 97 (23), 91(31).

| Elemental Analysis | | | |
|---|---|---|---|
| Calculated | 67.12% C; | 6.34% H; | 9.78% N. |
| Found | 66.92% C; | 6.52% H; | 9.52% N, |

### EXAMPLE 8

### Preparation of (D,L)-α-Acetamido-N-benzyl-2-benzofuranacetamide.

N-Acetyl-D,L-ethoxyglycine-N-benzylamide (1.00 g, 4 mmol) was suspended in anhydrous ethyl ether (30 mL) and then boron trifluoride etherate (0.91 g, 6.3 mmol) was rapidly added, and the resulting solution was stirred for 15 min. The benzofuran (1.89 g, 16 mmol) was then added and the reaction was stirred at room temperature (3 d). The reaction mixture was poured into an ice-cold saturated aqueous solution of NaHCO₃, and then the mixture was maintained at this temperature for an additional 15 min. The mixture was extracted with ethyl acetate (2 x), and the organic layers were combined, dried (Na₂SO₄) and evaporated in vacuo. The residue was purified by flash chromatography (100% chloroform, then 99:1 chloroform/methanol) to yield the desired product.
Yield: 0.43 g (33%).
R_{f} 0.30 (98:2 chloroform/methanol).
mp 195-196 °C;
¹H NMR (DMSO-d₆) δ 1.94 (s, CH₃CO), 4.34 (d, *J* = 5.7 Hz, CH₂), 5.77 (d, *J* = 8.1 Hz, CH), 7.24-7.32 (m, C_{3'}H, C_{5'}H, C_{6'}H, Ph), 7.54 (d, *J* = 7.0 Hz, C_{4'}H or C_{7'}H), 7.62 (d, *J* = 7.0 Hz, C_{4'}H or C_{7'}H), 8.74 (d, *J* = 8.1 Hz, NH), 8.86 (t, *J* = 5.7 Hz, NH).
¹³C NMR (DMSO-d₆) 22.27 (CH₃CO), 42.30 (CH₂), 51.22 (CH), 104.34 (C_{3'}), 110.90 (C_{7'}), 121.05 (C_{4'}), 122.90 (C_{5'}), 124.28 (C_{6'}), 126.73 (C_{3'a}) 127.01 (2C_{2"} or 2C_{3"}), 127.69 (2C_{2"} or 2C_{3"}), 128.14 (C_{4"}), 138.87 (C_{1"}), 154,10 (C_{7'a}), 154.30 (C_{2'}), 167.40 (CH₃CO), 169.26 (CONH) ppm.
IR (KBr) 3230, 1625 (br), 1520 (br), 1440, 1090, 1085, 890, 735, 690 cm⁻¹;
Mass spectrum, m/e (relative intensity) 322 (5), 279 (1), 264 (1), 234 (1), 215 (5), 189 (45), 146 (100), 130 (11), 118 (7), 91 (87), 65 (16).
High resolution mass spectrum,
Calcd for C₁₉H₁₈N₂O₃ 322.1317.
Found 322.1318.

### EXAMPLE 9

### Preparation of (D,L)-α-Acetamido-N-benzyl-2-benzo[b]thiopheneacetamide.

N-Acetyl-D,L-ethoxyglycine-N-benzylamide (1.00 g, 4 mmol) was suspended in anhydrous ethyl ether (15 mL) and then boron trifluoride etherate (0.91 g, 6.3 mmol) was rapidly added, and the resulting solution was stirred for 15 min. The benzo[b]thiophene (2.14 g, 16 mmol) was then added and the reaction was stirred at room temperature (3 d). The solution was poured into an ice-cold saturated aqueous solution of NaHCO₃, and then stirred for 15 min at 0 °C. The mixture was extracted with ethyl acetate (2 x), and the organic layers were combined, dried (Na₂SO₄) and evaporated in vacuo to give an orange oil. The oil was triturated with ethyl ether to yield a crystalline product which was filtered and further purified by flash chromatography (99:1 chloroform/methanol) to give the desired product.
Yield: 0.06 g (4%).
R_{f} 0.32 (99:1 chloroform/methanol).
mp 226.227 °C.
¹H NMR (DMSO-d₆) δ 1.94 (s, CH₃CO), 4.34 (d, *J* = 5.7 Hz, CH₂), 5.86 (d, *J* 8.1 Hz, CH), 7.20-7.38 (m, C_{3'}H, C_{6'}H. C_{7'}H, Ph), 7.77-7.80 (m, C_{4'}H or C_{5'}H), 7.89-7.93 (m, C_{4'}H or C_{5'}H), 8.76 (d, *J* = 8.1 Hz, NH), 8.97 (t, *J* = 5.7 Hz, NH).
¹³C NMR (DMSO-d₆) 22.34 (CH₃CO), 42.38 (CH₂), 52.70 (CH), 122.15 (C_{4'} or C_{7'}), 122.32 (C_{4'} or C_{7'}), 123.45 (C_{3'}), 124.37 (C_{5'} or C_{6'}), 124.41 (C_{5'} or C_{6'}), 126.89 (C_{4"}), 127.27 (2C_{2"} or 2C_{3"}), 128.27 (2C_{2"} or 2 C_{3"}), 138.84 (C_{3'a} or C_{7'a}), 138.95 (C_{3'a} or C_{7'a}). 142.58 (C_{1'}), 168.65 (CH₃CO), 169.12 (CONH) ppm. [A distinct signal for the C_{2'} carbon was not detected and is presumed to coincide with the C_{1'} carbon at 142.58 ppm.].
IR (KBr) 3240, 1610 (br), 1510 (br), 1420, 1360, 1215, 1085, 885, 730, 710, 685 cm⁻¹.
Mass spectrum, m/e (relative intensity) 338 (8), 295 (2), 205 (76), 162 (100), 135 (22), 108 (12), 91 (59).

| Elemental Analysis: | | | |
|---|---|---|---|
| Calculated | 67.43% C; | 5.36% H; | 8.28% N. |
| Found | 67.21% C; | 5.37 %H; | 8.12% N. |

### EXAMPLE 10

### Preparation of (D,L)-α-Acetamido-N-benzyl-3-indoleacetamide. (reference compound).

N-Acetyl-D,L-ethoxyglycine-N-benzylamide (0.69 g, 2.75 mmol) was suspended in anhydrous ethyl ether ( 20 mL) and then boron trifluoride etherate (0.63 g, 4.40 mmol) was rapidly added, and the resulting solution was stirred for 15 min. The indole (1.30 g, 11.00 mmol) was then added and the reaction was stirred at room temperature ( 22 h). Petroleum ether (35-60 °C) was added to the reaction, and the resulting semisolid material filtered, and washed with petroleum ether (35-60 °C). Purification of the reaction mixture was accomplished by flash chromatography (98:2 chloroform/methanol) to produce the title compound as a white solid.
Yield: 0.25 g (18%).
R_{f} 0.14 (93:5 chloroform/methanol)
mp 213-214 °C.
¹H NMR (DMSO-d₆) δ 1.90 (s, CH₃CO), 4.36 (d, *J* =6.0 Hz, CH₂), 5.72 (d, *J* = 7.2 Hz, CH), 6.90-7.37 (m, Ph, C_{2'}H), 7.02 (dd, *J* = 7.5 Hz, *J* = 7.5 Hz, C_{5'}H or C_{6'}H), 7.12 (dd, *J* = 7.5 Hz, *J* = 7.5 Hz, C_{5'}H or C_{6'}H), 7.39 (d, *J* = 7.5 Hz, C_{4'}H or C_{7'}H), 7.65 (d, *J* = 7.5 Hz, C_{4'}H or C_{7'}H), 7.86 (d, *J* = 7.2 Hz, NHCH), 8.13 (t, *J* = 6.0 Hz, NHCH₂), 10.30-10.80 (br s, NH).
¹³C NMR (DMSO-d₆) 22.32 (CH₃CO), 42.23 (CH₂), 49.98 (CH), 111.51 (C_{7'}), 112.08 (C_{3'}), 118.76 (C_{4'} or C_{6'}), 119.24 (C_{4'} or C_{6'}), 121.37 (C_{5'}), 123.94 (C_{2'}), 126.58 (C_{3'a}), 126.71 (C_{4"}), 127.33 (2C_{2"} or 2C_{3"}), 128.18 (2C_{2"} or 2C_{3"}), 136.28 (C_{7'a}), 139.44 (C_{1"}), 169.13 (CH₃CO), 170.81 (CONH) ppm.
IR (KBr) 3260, 1610 (br), 1515 (br), 1450, 1420, 1370, 1350, 1235, 1095, 895, 735, 715, 695, 600 cm⁻¹.
Mass spectrum, m/e (relative intensity) 321 (5), 278 (1), 264 (1), 233 (1), 214 (6), 187 (85), 171 (3), 145 (100), 118 (18), 91 (39).

| Elemental Analysis: | | | |
|---|---|---|---|
| Calculated | 71.01% C; | 5.96% H; | 13.06% N. |
| Found | 70.87% C; | 6.15% H; | 12.78% N. |

### EXAMPLE 11

### Preparation of (D,L)-α-Acetamido-N-benzyl-2-(5-methylpyrrole)acetamide.

N-Acetyl-D,L-ethoxyglycino-N-benzylamide (2.00 g, 8 mmol) was suspended in anhydrous ethyl ether (175 mL), and then boron trifluoride etherate (1.38 g, 9.7 mmol) was added and the resulting solution stirred (15 min). The 2-methylpyrrole (0.85 g, 10 mmol) was then added and the reaction mixture was stirred under N₂ (6 d), during which time the color of the reaction mixture turned reddish brown and a dark-brown deposit formed at the bottom of the flask. The clear solution was decanted and treated with an aqueous saturated NaHCO₃ solution containing ice (100 mL) for 30 min. The aqueous reaction mixture was extracted with ethyl acetate (3 x 30 mL). The combined extracts were dried (Na₂SO₄) and the solvent removed in vacuo. The brown oily residue was purified by flash column chromatography using 98:2 chloroform/methanol as the eluent to yield the desired compound. The product was recrystallized from ethyl acetate/hexane to give a light yellow amorphous solid.
Yield 0.20 g (94%)
R_{f} 0.44 (95:5, chloroform/methanol).
mp 167-168 °C.
¹H NMR (DMSO-d₆) δ 1.87 (s, CH₃), 2.13 (s, COCH₃), 4.27 (br s, CH₂), 5.33 (d, J = 7.4 Hz, CH), 5.60 (s, C₄H), 5.77 (s, C₃H), 7.19-7.30 (m, 5 PhH), 8.22 (d, J = 7.4 Hz, NH), 8.45 (t, J = 5.5 Hz, NH), 10.38 (s, NH).
¹³C NMR (DMSO-d₆) 12.74 (CH₃), 22.49 (COCH₃), 42.11 (CH₂), 51.21 (CH), 105.09 (C₄), 106.0₇ (C₃), 126.16 (C₅), 126.64 (C_{4'}), 126.85 (C₂), 127.09 (2_{C2'} or 2C_{3'}), 128.17 (2C_{2'} or 2C_{3'}), 139.33 (C_{1'}), 168.88 (COCH₃), 169.79 (CONH) ppm.
IR (KBr) 3250, 1630, 1520, 1420, 1360, 1300, 1260, 1230, 1160, 1110, 1020 cm⁻¹.
Mass spectrum, m/e (relative intensity) 285 (M⁺, 10), 178 (20), 152 (24), 151 (100), 110 (12), 109 (93), 108 (22), 107 (25), 94 (16), 91 (43).

| Elemental Analysis: | | | |
|---|---|---|---|
| Calculated | 67.35% C; | 6.71% H; | 14.73% N. |
| Found | 67.57% C; | 6.90% H; | 14.52% N. |

### Synthesis of Unsubstituted and Substituted-α-Acetamido-N-benzyl-2-furanacetamides.

General Procedure. 4-Methylmorpholine (1 equiv) was added to a solution of α-acetamido-2-furanacetic acid (1 equiv) in dry tetrahydrofuran (75 mL/10 mmol) at -10 to -15 °C under N₂. After stirring (2 min), isobutyl chloroformate (1 equiv) was added leading to the precipitation of a white solid. The reaction was allowed to proceed for 2 additional minutes and then a solution of the substituted benzylamine (1 equiv) in tetrahydrofuran (10mL/10 mmol) was added over 5 min at -10 to -15 °C. The reaction mixture was allowed to stir at room temperature for 5 min and then the 4-methylmorpholine hydrochloride salt filtered. The organic layer was concentrated in vacuo, and the residue was triturated with ethyl acetate, and the remaining white solid filtered. Concentration of the ethyl acetate layer led to additional amounts of the white solid. The desired product was purified by either recrystallization, or flash chromatography of the combined solid material. Examples 12-19 were prepared according to this procedure.

### EXAMPLE 12

### (D,L)-α-Acetamido-N-benzyl-2-furanacetamide.

Using benzyl amine (0.27 g, 2.56 mmol) and racemic α-acetamido-2-furanacetic acid (0.47 g, 2.56 mmol) gave the desired compound. The product was recrystallized from ethyl acetate to give a white solid.
Yield: 0.46 g (65%)
R_{f} 0.30 (98:2 chloroform/methanol).
mp 177-178 °C.
¹H NMR (DMSO-d₆) δ 1.90 (s, CH₃), 4.31 (d, J = 6.0 Hz, CH₂), 5.58 (d, J = 8.1 Hz, CH), 6.27 - 6.33 (m, C₃H), 6.40 - 6.44 (m, C₄H), 7.20 - 7.36 (m, 5 PhH), 7.60 - 7.64 (m, C₅H), 8.57 (d, J = 8.1 Hz, NH), 8.73 (t, J = 6.0 Hz, NH).

### EXAMPLE 13

### (D,L)-α-Acetamido-N-(2-fluorobenzyl)-2-furanacetamide.

Using 2-fluorobenzylamine (1.13 g, 9.0 mmol) and racemic α-acetamido-2-furanacetic acid (1.50 g, 8.2 mmol) gave the desired product.
Yield: 1.20 g (50%).
R_{f} 0.36 (96:4 chloroform/methanol).
mp 193-195 °C (recrystallized from EtOAc).
¹H NMR (DMSO-d₆) δ 1.89 (s, COCH₃), 4.33 (d, J = 5.5 Hz, CH₂), 5.58 (d, J = 8.0 Hz, CH), 6.28 (s, C₄H), 6.29 (s, C₃H), 7.62 (s, C₅H), 7.13-7.35 (m, 4 ArH), 8.61 (d, J = 8.0 Hz, NH), 8.76 (t, J = 5.5 Hz, NH).
¹³C NMR (DMSO-d₆) 22.35 (COCH₃), 36.12 (d, J_{CF} = 6.6 Hz, CH₂), 50.88 (CH), 107.64 (C₄), 110.43 (C₃), 115.04 (d, J_{CF}= 21.4 Hz, C_{3'}), 124.29 (d, J_{CF} = 4.2 Hz, C_{5'}), 125.64 (d, J_{CF} = 15.0 Hz, C_{1'}), 128.94 (d, J_{CF} = 9.0 Hz, C_{4'} or C_{6'}), 129.27 (d, J_{CF} = 5.5 Hz, C₄' or C_{6'}), 142.66 (C₅), 151.07 (C₂), 159.99 (d, J_{CF} = 244.4 Hz, C_{2'}), 168.17 (COCH₃), 169.24 (CONH) ppm.
IR (KBr) 3270, 1630, 1520, 1440, 1360, 1220, 1180, 1140, 1100, 1000, 740 cm⁻¹.
Mass spectrum, m/e (relative intensity) 291 (M⁺+1, 3), 274 (2), 247(3), 165 (4), 145 (10), 139 (98), 128 (46), 126 (7), 110 (10), 109 (65), 97 (93), 96 (100).

| Elemental Analysis: | | | |
|---|---|---|---|
| Calculated | 62.02% C; | 5.21% H; | 9.65% N. |
| Found | 62.20% C; | 5.19% H; | 9.69% N. |

### EXAMPLE 14

### (D,L)-α-Acetamido-N-(3-fluorobenzyl)-2-furanacetamide.

Making use of 3-fluorobenzylamine (1.13 g, 9.0 mmol) and racemic α-acetamido-2-furanacetic acid (1.50 g, 8.2 mmol) gave the desired product.
Yield 1.90 g (80%).
R_{f} 0.30 (96:4 chloroform/methanol).
mp 163-165 °C (recrystallized from ethyl acetate).
¹H NMR (DMSO-d₆) δ 1.89 (s, COCH₃), 4.31 (d, J = 5.5 Hz, CH₂), 5.55 (d, J = 7.8 Hz, CH), 6.31 (s, C₄H), 6.42 (s, C₃H), 6.98-7.37 (m, 4 ArH). 7.62 (s, C₅H), 8.61 (d, J = 7.8 Hz, NH), 8.70 (t, J = 5.5 Hz, NH).
¹³C NMR (DMSO-d₆) 22.35 (COCH₃), 41.71 (CH₂), 51.01 (CH), 107.73 (C₄), 110.59 (C₃), 113.50 (d, J_{CF} = 21.6 Hz, C_{2'} or C_{4'}), 113.60 (d, J_{CF} = 22.3 Hz, C_{2'} or C_{4'}), 122.95 (br, C_{6'}), 130.18 (d, J_{CF} = 8.6 Hz, C_{5'}), 142.21 (d, J_{CF} = 7.5 Hz, C_{1'}), 142.66 (C₅), 151.03 (C₂), 162.28 (d, J_{CF} = 243.3 Hz, C_{3'}), 168.23 (COCH₃), 169.31 (CONH) ppm.
IR (KBr) 3230, 1630, 1540, 1440, 1360,1220, 1140, 1000, 730 cm⁻¹.
Mass spectrum, m/e (relative intensity) 290 (M⁺,71), 231 (7), 165 (18), 140 (23), 139 (100), 126 (16), 109 (6), 97 (118), 96 (100), 96 (30).

| Elemental Analysis: | | | |
|---|---|---|---|
| Calculated | 62.02% C; | 5.21% H; | 9.65% N. |
| Found | 61.97% C; | 5.35% H; | 9.53% N. |

### EXAMPLE 15

### (D,L)-α-Acetamido-N-(4-fluorobenzyl)-2-furanacetamide.

Using racemic α-acetamido-2-furanacetic acid (1.50 g, 8.2 mmol) and 4-fluorobenzylamine (1.13 g, 9.0 mmol) gave the desired product.
Yield 2.10 g (88%).
R_{f} 0.30 (96:4 chlorofonn/methanol).
mp 188-190 °C (recrystallized from ethyl acetate).
¹H NMR (DMSO-d₆) δ 1.88 (s, COCH₃), 4.27 (d, J = 5.5 Hz, CH₂), 5.55 (d, J = 8.0 Hz, CH), 6.27 (s, 1H), .6.41 (s, 1H), 7.09-7.15 (m, 2ArH), 7.12-7.27 (m, 2 ArH), 7.61 (s, 1H), 8.58 (d, J = 8.0 Hz, NH), 8.75 (t, J = 5.5 Hz, NH).
¹³C NMR (DMSO-d₆) 22.28 (COCH₃), 41.51 (CH₂), 50.87 (CH), 107.52 (C₄). 110.46 (C₃), 114.90 (d, J_{CF} = 21.1 Hz, C_{3'}), 129.48 (d, J_{CF} = 8.3 Hz, C_{2'}), 135.23 (d, J_{CF} = 3.2 Hz, C_{1'}), 142.53 (C₅), 151.08 (C₂), 161.12 (d, J_{CF} = 242.2 Hz, C_{4'}), 167.95 (COCH₃), 169.13 (CONH) ppm.
IR (KBr) 3230, 1620, 1500, 1360, 1320, 1260, 1210, 1140, 1000, 820, 780, 730 cm⁻¹.
Mass spectrum, m/e (relative intensity) 291 (M⁺+1, 4), 165 (4), 140 (9), 139 (92), 138 (52), 124 (6), 109 (71), 97 (60), 96 (100).

| Elemental Analysis: | | | |
|---|---|---|---|
| Calculated | 62.02% C; | 5.21% H; | 9.65% N. |
| Found | 61.76% C; | 5.41% H; | 9.43% N. |

### EXAMPLE 16

### (D,L)-α-Acetamido-N-(2,5-fluorobenzyl)-2-furanacetamide.

Using 2,6-difluorobenzylamine (1.30 g, 9.0 mmol) and racemic α-acetamido-2-furanacetic acid (1.50 g, 8.2 mmol) gave the desired product.
Yield 1.60 g (64%).
R_{f} 0.38 (96:4 chloroform/methanol).
mp 177-178 °C (recrystallized from ethyl acetate).
¹H NMR (DMSO-d₆) δ 1.89 (s, COCH₃), 4.31 (d, J = 5.5 Hz, CH₂), 5.55 (d, J = 7.7 Hz, CH), 6.32 (s, C₄H), 6.43,(s, C₃H), 7.22-7.25 (m, 3 ArH), 7.62 (s, C₅H), 8.62 (d, J = 7.7 Hz, NH), 8.78 (t, J= 5.5 Hz, NH).
¹³C NMR (DMSO-d₆) 22.30 (COCH₃), 35.98 (d, J_{CF} = 5.8 Hz, CH₂), 51.02 (CH), 107.81 (C₄), 110.58 (C₃), 115.06 (dd, J_{CF} = 19.5, 25.6 Hz, C_{3'} or C_{6'}), 115.16 (dd, J_{CF} 15.6, 24.7 Hz, C_{3'} or C_{6'}), 116.52 (dd, J_{CF} = 10.1, 23.9 Hz, C_{4'}), 127.98 (dd, J_{CF} = 9.2, 17.7 Hz, C_{1'}), 142.69 (C₅), 150.78 (C₂), 155.89 (d, J_{CF} = 239.0 Hz, C_{2'} or C_{5'}), 158.18 (d, J_{CF} = 238.8 Hz, C_{2'} or C_{5'}), 168.38 (COCH₃), 169.35 (CONH) ppm.
IR (KBr) 3230, 1620, 1520, 1480, 1360, 1260, 1230, 1180, 1140, 1000, 860, 810, 730, 710 cm⁻¹.
Mass spectrum, m/e (relative intensity) 309 (M⁺+1, 1), 266 (1), 222(1), 165 (5), 140 (5), 139 (61), 138 (36), 127 (37), 97 (44), 96 (100).

| Elemental Analysis: | | | |
|---|---|---|---|
| Calculated | 58.44% C; | 4.58% H; | 9.09% N. |
| Found | 58.68% C; | 4.69% H; | 8.87% N, |

### EXAMPLE 17

### (D,L)-α-Acetamido-N-(2,6-difluorobenzyl)-2-furanacetamide.

Making use of 2,6-difluorobenzylamine (1.30 g, 9.0 mmol) and racemic α-acetamido-2-furanacetic acid (1.50 g, 8.2 mmol) gave the desired product.
Yield 1.90 g (73%).
mp 237-239 °C (recrystallized from ethanol).
¹H NMR (DMSO-d₆) δ 1.86 (COCH₃), 4.33 (d, J = 4.5 Hz, CH₂), 5.53 (d, J = 8.3 Hz, CH), 6.17 (s, C₄H), 6.38 (s, C₃H), 7.05-7.10 (m, 2 ArH), 7.36-7.41 (m, 1 ArH), 7.60 (s, C₅H). 8.52 (d, J = 8.3 Hz, NH). 8.66 (t, J = 4.5 Hz, NH).
¹³C NMR (DMSO-d₆) δ 22.33 (COCH₃), 30.74 (t, J_{CF} = 4.4 Hz, CH₂), 50.48 (CH), 107.24 (C₄), 110.40 (C₃), 111.61 (dd, J_{CF} = 8.0, 25.1 Hz, C_{3'}, C_{5'}) 113.67 (t, J_{CF} = 10.5 Hz, C_{1'}), 129.98 (t, J_{CF} = 10.5 Hz, C_{4'}), 142.50 (C₅), 151.23 (C₂), 180.93 (J, J_{CF} = 248.1, C_{2'} or C_{6'}), 161.10 (d, J_{CF} = 248.1 Hz, C_{2'} or C_{6'}), 167.59 (COCH₃), 169.00 (CONH) ppm.
IR (KBr) 3230, 1620, 1530, 1460, 1360, 1320, 1260, 1220, 1160, 1140, 1030, 1000, 820, 780, 750, 740, 710 cm⁻¹.
Mass spectrum, m/e (relative intensity) 309 (M⁺+1, 4), 265 (2), 165 (4), 147 (7), 140 (8), 139 (87), 138 (36), 127 (54), 97 (58), 96 (100).

| Elemental Analysis: | | | |
|---|---|---|---|
| Calculated | 58.44% C; | 4.58% H; | 9.09% N. |
| Found | 58.62% C; | 4.74% H; | 8.99% N. |

### EXAMPLE 18

### (D)-(+)-α-Acetamido-N-benzyl-2-furanacetamide.

Starting with D-α-acetamido-2-furanacetic acid (2.45 g, 13.38 mmol) and benzylamine (1.43 g, 13.38 mmol), the desired product was obtained,
Yield: 2.54 g (70%) The product was further recrystallized from ethyl acetate to give the title compound.
Yield: 2.30 g
mp 196-197 °C.
[α]²⁶_{D}[c = 1, MeOH] = -78.3°. Addition of R(-)-mandelic acid to a CDCl₃ solution of the product gave only one signal for the acetamide methyl protons.
Mass spectrum, m/e (relative intensity) 272 (M⁺, 2), 184 (2), 165 (2), 140 (8), 139 (88), 138 (34), 97 (46), 96 (100), 91 (63).

| Elemental Analysis: | | | |
|---|---|---|---|
| Calculated | 66.16% C; | 5.92% H; | 10.29% N, |
| Found | 66.09% C; | 6.01% H; | 10.38% N. |

### EXAMPLE 19

### (L)-(+)-α-Acetamido-N-benzyl-2-furanacetamide.

Using L-α-acetamido-2-furanacetic acid (2.83 g, 15.46 mmol) and benzylamine (1.65 g, 15.46 mmol) gave 3.80 g of the enriched desired product. ¹H NMR analysis with R(-)-mandelic acid showed that it was greater than 80% enriched in the title compound. The pure L-enantiomcr was obtained by recrystallization from absolute ethanol.
Yield: 1.60 g.
mp 196-197 °C.
[α]²⁶_{D}[c = 1, MeOH] = +79.0°.
Mass spectrum, m/e (relative intensity) 273 (M⁺ + 1, 3), 229 (2), 214 (2), 184 (1), 165 (7), 157 (4), 140 (33), 139 (100), 138 (95), 97 (98), 96 (100), 91 (98).

| Elemental Analysis: | | | |
|---|---|---|---|
| Calculated | 66.16% C; | 5.92% H; | 10.29% N. |
| Found | 65.89% C; | 5.86% H; | 10.42% N. |

### EXAMPLE 20

### Resolution of (D,L)-α-Acetamido-2-furanacetic acid Using (R)-(+)-α-Methylbenzylamine and (S )-(-)-α-Methylbenzylamine.

(R)-(+)-α-Methylbenzylamine (13.22 g, 0.11 mol) was added to an absolute ethanol solution (550 mL) of racemic α-acetamido-2-furanacetic acid (20.00 g, 0.11 mol). The resulting solution was cooled in the freezer overnight. The white precipitate (12.00 g) which separated upon cooling was filtered, and. the mother liquid evaporated to give a salt which was later used for obtaining L-α-acetamido-2-furanacetic acid. The initial salt was recrystallized (3 x) from absolute ethanol to yield 4.00 g of the pure diasterometic salt.
mp 173-175°C,
[α]²⁶_{D}[c=1, MeOH]=-108°.

| Elemental Analysis | | | |
|---|---|---|---|
| Calculated | 63.14% C; | 6.62% H; | 9.21% N. |
| Found | 63.19% C; | 6.62% H; | 9.12% N. |

The purified salt was treated with 5% aqueous NH₄OH solution, extracted with ethyl ether (3 x 50 mL), and then acidified with a 8.5% aqueous solution of H₃PO₄ and then extracted with ethyl acetate (3 x 100 mL) to yield 2.45 g(25%) of D-α-acetamido- 2-furanacetic acid.
mp 169-171°C.
[α]²⁶_{D}[c=1, MeOH] =-184.2°.

| Elemental Analysis: | | | |
|---|---|---|---|
| Calculated | 52.46% C; | 4.95% H; | 7.65% N. |
| Found | 52.17° C; | 4.89% H; | 7.56% N. |

The salt obtained after evaporation of the main mother liquor was hydrolysed with 5% aqueous NH₄OH solution to give 10.10 g of the enriched L-α-acecamido-2-furanacetic acid [[α]²⁶D[c=1, MeOH] = +47.7°]. (S)-(-)-methylbenzylamine (6.70 g, 0.055 mol) was added to a solution of enriched L-α-acecamido-2-fucanacetic acid (10.10 g, 0.055 mol) in absolute ethanol (275 mL). The white precipitate of the diasteroemeric salt (8.10 g) that separated upon cooling the solution in the freezer (1 h) was filtered. The salt was recrystallized from absolute ethanol (3 x) to yield 3.00g of the salt,
mp 172-174 °C.
[α]²⁶D[c=1, MeOH]= +106°.

| Elemental Analysis: | | | |
|---|---|---|---|
| Calculated | 63.14% C; | 6.62% H; | 9.21% N. |
| Found | 63.18% C; | 6.47% H; | 9.00% N. |

The salt from the third recrystallization was treated with a 5% aqueous NH₄OH solution and extracted with ethyl ether (3 x 50 mL), and then acidified with a 8.5% aqueous solution of H₃PO₄, and then extracted with ethyl acetate (3 x 100 mL) to give 1.63g (32%) of L-α-acetamido-2-furanacetic acid.
mp 169-171°C.
[α]²⁶D[c=1, MeOH]= +182°.

### EXAMPLE 21

### Enzymatic Separation of D(-)α-acetamido-2-furanacetic acid from DL (±)α-acetamido-2-furanacetic acid.

DL (±)α-acetamido-2-furanacetic acid (2.00 g/ 10.9 mmol) was suspended in deionized H₂O (600mL). An aqueous solution of LiOH (1N) was added to this suspension dropwise until all of the acid had dissolved and the pH was 7.2-Acylase 1, Grade II (20 mg, activity = 900 units/mg, Sigma Chemical Company, Cat. No. A 8376) was then added to the above solution and the mixture stirred at 34-37°C (41h). The suspension was then cooled to room temperature and acidified to pH 1.5 with aqueous 1N HCl. The suspended material was filtered, and the filtrate was saturated with solid NaCl, and then extracted with ethyl acetate (3x250 mL). The combined ethyl acetate extracts was dried (Na₂SO₄). The solvent was removed in vacuo and the residue was triturated with ethyl acetate (10mL). The white solid (0.75 g) that remained was filtered and was pure D(-)α-acetamido-2-furanacetic acid; mp 168-169°C, mixed mp with an authentic sample 168-169°C; [α]_{D}²⁶ [c=1, MeOH] =-184.3°.

### EXAMPLE 22

### Preparation of D,L-α-Acetamido-2-furanacetic Acid.

An ethereal solution of ZnCl₂( 1 N, 28 mL, 0.028 mol) was added to a stirred solution of ethyl acetamido-2-bromoacetate (4.40 g, 0.019 mol) and furan (11.23 g, 0.165 mol) in dry tetrahydrofuran (100 mL), and allowed to stir at room temperature (5 h). The mixture was then treated with H₂O (50mL), the organic phase separated, and the aqueous layer extracted with CH₂Cl₂ (2 x 100 mL). The organic layers were combined, dried (Na₂SO₄) and the volatile materials were removed by distillation in vacuo to give approximately 4.00 g (97%) of light-brown semi-solid material. TLC analysis showed a major spot at R_{f} 0.30 (99:1 chloroform/methanol). The desired compound, D,L-ethyl α-acetamido-2-furanacetate, was purified by flash column chromatography on silica gel using 99:1 chloroform/methanol as the eluent to give 3.60 g (87%) of a beige solid.
mp 68-70 °C.

D, L-Ethyl α-acetamido-2-furanacetate (4.00 g, 19 mmol) was dissolved in 90:10 ethanol/water (150 mL) and then KOH (2.00 g, 35 mmol) was added and the resulting solution stirred at room temperature (48 h). The reaction was concentrated in vacuo and the residue diluted with H₂O and then washed with ethyl ether (3 x 50 mL). The aqueous layer was then made acidic with a 8.5% aqueous solution of H₃PO₄ and extracted with ethyl acetate (3 x 150 mL). The organic layers were combined, dried (Na₂SO₄), evaporated to dryness in vacuo to give the desired compound.
Yield: 2.65 g (76%).
R_{f} 0.37 (8:1:1 isopropanol/NH₄OH/H₂O).
mp 172-174 °C.

### EXAMPLE 23

### Synthesis of (D,L)-2-Acetamido-4-pentenoic Acid-N-benzylamide.

4-Methylmorpholine (0.55 g, 5.40 mmol) was added to a stirred solution of 2-acetamido-4-pentenoic acid (0.81 g, 5.18 mmol) in dry tetrahydrofuran (60 mL) at -10 to -15 °C under N₂. After stirring (2 min), isobutyl chloroformate (0.75 g, 5.70 mmol) was added leading to the precipitation of a white solid. The reaction was allowed to proceed for 2 additional minutes and then a solution of benzylamine (0.61 g, 5.70 mmol) in tetrahydrofuran (10 mL) was added slowly at -10 to -15 °C. After stirring (5 min) at room temperature, the insoluble salt was removed by filtration. The filtrate was evaporated to dryness and the residue was triturated with ethyl acetate, and the remaining white solid was filtered to yield the desired product.
Yield 0.81 g(64%).
R_{f} 0.36 (4% methanol/chloroform).
mp 118-120 °C (recrystallized from ethyl acetate/cyclohexane).
¹H NMR (DMSO-d₆) δ 1.83 (s, COCH₃), 2.22-2.49 (m, CH₂CH=CH₂), 4.26 (d, J = 5.3 Hz, CH₂ Ph), 4.25-4.33 (m, CH), 4.99-5.09 (m, CH₂CH=CH₂), 7.21-7.29 (m, 5 PhH). 8.05 (d, J = 7.6 Hz, NH), 8.46 (br s, NH).
¹³C NMR (DMSO-d₆) 22.41 (COCH₃), 36.24 (CH₂CH=CH₂), 41.91 (CH₂Ph), 52.20 (CH), 117.15 (CH₂CH=CH₂), 126.54 (C_{4'}), 126.99 (2C_{2'} or 2C_{3'}), 128.04 (2C_{2'} or 2C_{3'}), 134.25 (CH₂CH=CH₂), 139.22 (C_{1'}), 169.02 (COCH₃), 170.96 (CONH) ppm.
Mass spectrum, m/e (relative intensity) 246 (M⁺, 4), 205 (4), 163 (15), 140 (8), 106 (33), 91 (77), 70 (100).

| Elemental Analysis: | | | |
|---|---|---|---|
| Calculated | 68.27%C; | 7.37% H; | 11.37% N. |
| Found | 68.55% C; | 7.31% H; | 11.48% N. |

### EXAMPLE 24

### Synthesis of (D,L)-2-Acetamido-N-benzyl-2-(1-morpholine)acetamide. (reference compound)

A mixture of ethyl 2-acetamido-2-(1-morpholine)acetate (0.59 g, 2.56 mmol), benzylamine (0.28 g, 2.82 mmol) and sodium cyanide (0.01 g, 0.26 mmol) in methanol (5 mL) was stirred at 50-55 °C for 18 hr. The solvent was removed in vacuo and the residue triturated with ethyl acetate (5 mL). The white solid (0.35 g) that remained was collected by filtration and identified as the desired compound. The filtrate was concentrated and the residue purified by flash column chromatography (2% methanol/chloroform) on SiO₂. The initial fractions gave a trace amount (0.09 g) of (D,L)-2-acetamido-N-benzyl-2-(N-benzylamine)acetamide. Continued elution gave additional amounts (0.20 g) of the title compound.
*(D,L)-2-Acetamido-N-benzyl-2-(N-benzylamine)acetamide:*
Yield: 0.09 g (11 %).
mp 135-138 °C.
¹H NMR (DMSO-d₆) δ 1.83 (s, COCH₃), 3.56 (d, J = 13.6 Hz, NHCH), 3:66 (d, J = 13.6 Hz, NHCH), 4.23 (d, J = 5.4 Hz, CH₂), 4.89 (d, J = 8.0 Hz, CH), 7.05-7.38 (m, 10 PhH), 8.20 (d, J = 8.0 Hz, NH), 8.51 (t, J = 5.4 Hz, NH).
¹³C NMR (DMSO-d₆) 22.63 (COCH₃), 42.11 (CH₂), 48.57 (NHCH₂), 64.41 (CH), 126.65 (C₄), 126.70 (C_{4'}), 127.13, 128.00, 128.13, 128.22, 139.24 (C₁ or C_{1'}), 140.12 (C₁ or C_{1'}), 169.61 (COCH₃), 169.90 (CONH) ppm.
*(D,L)-2-Acetamido-N-benzyl-2-(1-morpholine)acetamide.*
Yield: 0.48 g (64%).
R_{f} 0.35 (4% methanol/chloroform).
mp 171-172° (recrystallized from ethyl acetate).
¹H NMR (DMSO-d₆) δ 1.86 (s, COCH₃), 2.30-2.40 (m, CH₂NCH₂), 3.51 (br s, CH₂OCH₂), 4.18-4.33 (m, CH₂), 5.07 (d, J = 8.9 Hz, CH), 7.18-7.25 (m, 5 PhH), 8.23 (d, J = 8.9 Hz, NH), 8.58 (br s, NH).
¹³C (DMSO-d₆) 22.39 (COCH₃), 42.20 (CH₂), 48.43 (CH₂NCH₂), 66.03 (CH), 69.24 (CH₂OCH₂), 126.76 (C_{4'}), 127.13 (2C_{2'} or 2C_{3'}), 128:23 (2C_{2'} or 2_{C3'}), 139.42 (C_{1'}), 168.02 (COCH₃), 170.20 (CONH) ppm.
Mass spectrum m/e (relative intensity) 292 (M⁺+1, 1), 233 (8), 158 (19), 157 (100), 116 (26), 115 (100), 106 (29), 91 (72).

| Elemental Analysis: | | | |
|---|---|---|---|
| Calculated | 61.84% C; | 7.26% H; | 14.42% N. |
| Found | 61.67 % C; | 7.10% H; | 14.14% N. |

### EXAMPLE 25

### Synthesis of (D,L)-2-Acetamido-N-benzyl-2-(N-anilino)acetamide.

A mixture of ethyl 2-acetamido-2-(N-anilino)acetate (2.00 g, 8.47 mmol), benzylamine (1.09 g, 10.00 mmol), and sodium cyanide (0.04 g, 0.84 mmol) in methanol (20 mL) was heated with stirring at 45-50 °C (18 h). The white solid which separated during the course of the reaction was filtered and purified by recrystallization from absolute ethanol to give the desired compound.
Yield: 1.10 g (72%).
mp 183-185 °C.
¹H NMR (DMSO-d₆) δ 1.84 (COCH₃), 4.31 (d, J = 5.8 Hz, CH₂), 5.67 (t, J = 8.1 Hz, CH), 6.04 (d, J = 8.1 Hz, NHPh), 6.59-6.64 (m, 1 PhH), 6.70-6.72 (m, 2 PhH), 7.06-7.11 (m, 2 PhH), 7.20-7.33 (m, 5 PhH), 8.41 (d, J = 8.1 Hz, NH), 8.72 (t, J = 5.8 Hz, NH)
¹³C NMR (DMSO-d₆) 22.46 (COCH₃), 42.25 (CH₂), 60.42 (CH), 113.21 (2C₂), 117.22 (C₄), 126.72 (C_{4'}), 127.16 (2C_{2'} or 2C_{3'}), 128.18 (2C_{2'} or 2C_{3'}), 128.77 (2C₃), 138.99 (C_{1'}), 145.88 (C₁), 168.65 (COCH₃), 169.70 (CONH) ppm.
Mass spectrum m/e (relative intensity) 297 (M⁺, 2), 239 (7), 177 (3), 164 (28), 163 (100), 122 (20), 121 (100), 106 (47), 104 (65), 93 (63), 91 (77), 77 (49).

| Elemental Analysis: | | | |
|---|---|---|---|
| Calculated | 68.67% C; | 6.44% H; | 14.13% N. |
| Found | 68.94% C; | 6.42% H; | 13.92% N. |

### EXAMPLE 26

### Synthesis of (D,L)-2-Acetamido-N-benzyl-2-(methylamino)acetamide.

A mixture of ethyl 2-acetamido-2-(methylamino)acetate (1.50 g, 8.63 mmol), benzylamine (1.11 g, 10.35 mmol) and sodium cyanide (0.04 g, 0.82 mmol) in methanol (20 mL) was stirred at 50-55 °C (18 h). The solvent was removed in vacuo and then the residue was purified by flash column chromatography on SiO₂ using 2% methanol/chloroform as the eluent to yield the desired compound.
Yield: 1.00 g (49%).
R_{f} 0.33 (3% methanol/chloroform).
mp 115-117 °C (recrystallized from ethyl acetate/petroleum ether).
¹H NMR (DMSO-d₆) δ 1.87 (s, COCH₃), 2.18 (s, NHCH₃), 4.20-4.29 (m, CH₂), 4.87 (d, J = 7.9 Hz, CH), 7.24-7.35 (m, 5 PhH), 8.14 (d, J = 7.9 Hz, NH), 8.55 (br s, NH).
¹³C NMR (DMSO-d₆) 22.52 (COCH₃), 31.37 (NHCH₃), 42.04 (CH₂), 65.99 (CH), 126.68 (C_{4'}), 127.12 (2C_{2'} or 2C_{3'}), 128.18 (2C_{2'} or 2C_{3'}), 139.28 (C_{1'}), 169.51 (COCH₃), 169.83 (CONH) ppm.

| Elemental Analysis: | | | |
|---|---|---|---|
| Calculated | 61.26% C; | 7.28% H; | 17.86% N. |
| Found | 61.12% C; | 7.01%H; | 17.74% N. |

### EXAMPLE 27A

### Synthesis of (D,L)-Ethyl 2-acetamido-2-(ethylamino)acetate.

A cold (-78 °C) solution of ethyl 2-acetamido-2-bromoacetate (2.10 g. 9.38 mmol) in dry tetrahydrofuran (80 mL) was added slowly into a cooled (-78 °C) tetrahydrofuran (20 mL) solution of methylamine (1.40 g, 31.04 mmol) over a period of 20 min. The reaction was stirred at -78 °C (1 h), and then at room temperature (1 h). The precipitated salt was filtered and the filtrate concentrated. The residue was purified by flash column chromatography on SiO₂ using 3% methanol/chloroform as the eluent to yield, the desired compound as a light yellow oil.
Yield: 0.90 (51%).
R_{f} 0.36 (4% methanol/chloroform).
¹H NMR (CDCl₃) 0.93 (t, J = 6.7 Hz, NHCH₂CH₃), 1.12 (t, J = 6.8 Hz, OCH₂CH₃), 1.87 (s, COCH₃), 2.48 (q, J = 6.7 Hz, NHCH₂CH₃), 4.05 (q, J = 6.8 Hz, OCH₂CH₃), 5.05 (d, J = 7.1 Hz, CH), 7.09 (d, J = 7.1 Hz, NH).
¹³C NMR (CDCl₃) 13.64 (NHCH₂CH₃), 14.55 (OCH₂CH₃), 22.53 (COCH₃), 39.06 (NHCH₂CH₃). 61.38 (CH), 64.14 (OCH₂CH₃), 170.09 (COCH₃), 170.20 (COOCH₂CH₃) ppm.

### EXAMPLE 27B

### Synthesis of (D,L)-2-Acetamido-N-benzyl-2-(ethylamino)acetamide.

A mixture of ethyl 2-acetamido-2-(ethylamino)acetate (0.90 g, 4.79 mmol), benzylamine (0.62 g, 5.75 mmol) and sodium cyanide (0.03 g, 0.51 mmol) in methanol (10 mL) was stirred at 50-55 °C (18 h). The solution was concentrated in vacuo, and the residue was purified by flash column chromatography on SiO₂ using 3% methanol/chloroform as the eluent to give the desired product as a white solid.
Yield: 0.35 g (29%).
R_{f} 0.34 (4% methanol/chloroform).
mp 123-125 °C (recrystallized from ethyl acetate/hexane).
¹H NMR (DMSO-d₆) δ 0.93 (t, J =, NHCH₂CH₃), 1.81 (s; COCH₃), 2.08 (br s, NHCH₂CH₃), 2.40-2.48 (m, NHCH₂CH₃), 4.22 (d, J = 5.5 Hz, CH₂), 4.90 (d, J = 7.8 Hz, CH), 7.20-7.27 (m, 5 PhH), 8.08 (d, J = 7.8 Hz, NH), 8.48 (br s, NH).
¹³C NMR (CDCl₃) 15.14 (NHCH₂CH₃), 22.97 (COCH₃), 37.65 (NHCH₂CH₃), 43.53 (CH₂), 65.68 (CH), 127.50 (C_{4'}), 127.44 (2C_{2'} or 2C_{3'}), 128.64 (2C_{2'} or 2C_{3'}), 137.73 (C_{1'}) 169.75 (COCH₃), 171.20 (CONH) ppm.

### EXAMPLE 28

Using the procedures described herein, the following examples are also prepared:
(D,L)α-Acetamido-N-benzyl-3-furanacetamide
(D,L)α-Acetamido-N-(2-fluorobenzyl)-3-furanacetamide
(D,L)α-Acetamido-N-(3-fluorobenzyl)-3-furanacetamide
(D,L)α-Acetamide-N-(4-fluorobenzyl)-3-furanacetamide
α-Acetamide-N-benzyl-2-aminoacetamide

### EXAMPLE 29

Using the procedure described herein the following compound can also be prepared: 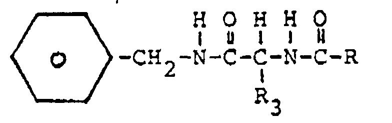 wherein R₃ is 2 pyridyl.

Pharmacology. The following compounds were'tested for anticonvulsant activity using male Carworth Farms #1 mice:
N-Acetyl-D,L-alanine-N'-benzylamide
N-Acetyl-D-alanine-N'-benzylamide
N-Acetyl-L-alanine-N'-benzylamide
N-Acetyl-D,L-phenylglycine-N'-methylamide
N-Acetylglycine-N-benzylamide
N-Acetyl-D,L-valine-N-benzylamide
N-Acetyl-D,L-phenylglycine-N-benzylamide
N-Acetyl-D-phenylglycine-N-benzylamide
N-Acetyl-L-phenylglycine-N-benzylamide
N-Acetyl-D,L-alanine-N-(3-methoxy)benzylamide
N-Trimethylacetyl-D,L-alanine-N-benzylamide
N-Acetyl-D,L-methionine-N-benzylamide
N-Acetyl-D,L-alanine-N'-(3-fluoro)benzylamide
D,L-α-Acetamido-N-benzyl-3-thiopheneacetamide
D,L-α-Acetamido-N-benzyl-2-thiopheneacetamide
D,L-α-Acetamido-N-benzyl-2-furanacetamide
D,L-α-Acetamido-N-benzyl-2-pyrroleacetamide
D,L-2-Acetamido-N-benzyl-2-ethoxyacetamide
D,L-α-Acetamido-N-benzyl-2-(5-methylfuran)acetamide
D,L-α-Acetamido-N-benzyl-2-benzofuranacetamide
D,L-α-Acetamido-N-benzyl-2-benzo[b]thiopheneacetamide
D,L-α-Acetamido-N-benzyl-2-(5-methylpyrrole)acetamide
D,L-α-Acetamido-N-(2-fluorobenzyl)-2-furanacetamide
D,L-α-Acetamido-N-(3-fluorobenzyl)-2-furanacetamide
D,L-α-Acetamido-N-(4-fluorobenzyl)-2-furanacetamide
D,L-α-Acetamido-N-(2,5-difluorobenzyl)-2-furanacetamide
D,L-α-Acetamido-N-(2,6-difluorobenzyl)-2-furanacetamide
D-(-)-α-Acetamido-N-benzyl-2-furanacetamide
L-(+)-α-Acetamido-N-benzyl-2-furanacetamide
D,L-2-Acetamido-4-pentenoic acid-N-benzylamide
D,L-2-Acetamido-N-benzyl-2-(1-morpholine)acetamide (reference compound)
D,L-2-Acetamido-N-benzyl-2-(N-anilino)acetamide
D,L-2-Acetamido-N-benzyl-2-(methylamino)acetamide
D,L-2-Acetamido-N-benzyl-2-(ethylamino)acetamide
D,L-2-Acetamido-N-benzyl-3-indoleacetamide (reference compound)

The compound was given in various dose levels (i.e., 10, 30, 100, 300 mg) and subsequently compared with phenytoin, phenobarbital, mephenytoin and phenacemide (See Table I). N-Acetyl-D,L-alanine-N'-benzylamide was tested at 600 mg/mL as well. Seizures were then artificially induced by either electroshock or pentylenetetrazole. Maximal electroshock seizures (MES) were elicited with a 60 cycle alternating current of 50 mA intensity (5-7 times that necessary to elicit minimal electroshock seizures) delivered for 0.2 sec via corneal electrodes. A drop of 0.9% saline was instilled in the eye prior to application of the electrodes so as to prevent the death of the animal. Protection in this test was defined as as the abolition of the hind limb tonic extension component of the seizure. The Subcutaneous Pentylenetetrazole (Metrazol^{R}) Seizure Threshold Test (sc Met) entailed the administration of 85 mg/kg of pentylenetetrazole as a 0.5% solution subcutaneously in the posterior midline. This amount of pentylenetetrazole was expected to produce seizures in greater than 95% of mice. The animal was observed for 30 minutes. Protection was defined as a failure to observe even a threshold seizure (a single episode of clonic spasms of at least 5 sec duration). The effects of the compounds on forced and spontaneous motor activity were evaluated in mice using the Rotorod Test (Tox) or, in some cases, as indicated herein, a horizontal screen assay (HS). The procedure for the rotorod test is described in J. Am. Pharm. Assoc., 46, 208-209 (1957).
In the rotorod test, the animal was placed on a one-inch diameter knurled plastic rod rotating at 6 rpm after the administration of the drug. Normal mice can remain on a rod rotating at this speed indefinitely. Neurologic toxicity was defined as the failure of the animal to remain on the rod for one minute. In the horizontal screen test, previously trained mice were dosed with the compound and placed individually on top of a square (13 cm X 13 cm) wire screen (no. 4 mesh) which was mounted on a metal rod. The rod was rotated 180°, and the number of mice that returned to the top of the screen was determined. Inability to climb to the top within one minute was defined as "neurological impairment". This procedure is described in Pharmacol. Biochem. Behav. 6, 351-353 (1977).

The dose effect behavior of the compounds was evaluated using the above-described procedures by the administration of varying dose levels, treating normally eight mice at each dose. Table I includes an evaluation of the Median Effective Dose (ED50) and the Median Toxic Dose (TD50) of representative compounds. Mice were tested with varying doses of the anticonvulsant to define the limits of complete protection (or toxicity) and no protection (or no toxicity), as well as three points in between these limits. The Median Effective Dose (ED50) was defined as the dose which produced the desired endpoint in 50% of the animals. The Median Toxicity Dose (TD50) was the dose which elicited evidence of minimal neurological toxicity in 50% of the animals. The results are given in Table I.

**TABLE I**

| Comparative Median Effective Dosage | | | |
|---|---|---|---|
| Compound | Tox TD50 mg/kg | MES ED50 mg/kg | sc Met ED50 mg/kg |
| (D,L)-α-Acetamido-N-benzyl-2-(5-methylfuran)acetamide | 75.4^{xx} | 19.2 (16.4-23.8)* | ≠ |
| | | | |
| (D,L)-α-Acetamido-N-benzyl-2-benzofuranacetamide | >100<300^{xx} | >100<300 | ≠ |
| | | | |
| (D,L)-α-Acetamido-N-benzyl-2-benzo[b]-thiopheneacetamide | >100<300^{xx} | >100<300 | ≠ |
| | | | |
| (D,L)-α-Acetamido-N-benzyl-2-(5-methylpyrrole)acetamide | x | 36.5 (30.6-57.1)* | ≠ |
| | | | |
| (D,L)-α-Acetamido-N-(2-fluorobenzyl)-2-furanacetamide | x | 40.0 | ≠ |
| | | | |
| (D,L)-α-Acetamido-N-(3-fluorobenzyl)-2-furanacetamide | 135.6 (114.9-161.8)^{xx} | 13.3 (11.5-15.3)* | ≠ |
| | | | |
| (D,L)-α-Acetamido-N-(4-fluorobenzyl)-2-furanacetamide | 144.4 (122.5-170.9)^{xx} | 12.7 (10.4-15.1)* | ≠ |
| | | | |
| (D,L)-α-Acetamido-N-(2,5-difluorobenzyl)-2-furanacetamide | x | 23.8 (20.2-28.4)* | ≠ |
| | | | |
| (D,L)-α-Acetamido-N-(2,6-difluorobenzyl)-2-furanacetamide | x | >25<100 | ≠ |
| | | | |
| (D,L)-α-Acetamido-N-benzyl-2-furanacetamide | 23.8^{xx} | 3.3 (2.8-3.9)* | ≠ |
| (D,L)-α-Acetamido-N-benzyl-2-furanacetamide | >300 | >100<300 | ≠ |
| | | | |
| (D,L)-2-Acetamido-4-pentenoic acid-N-benzylacetamide | x | 33.6 | ≠ |
| | | | |
| (D,L)-2-Acetamido-N-benzyl-2-(1-morpholine)acetamide reference compound | x | >30<100 | ≠ |
| | | | |
| (D,L)-2-Acetamido-N-benzyl-2-(N-anilino)acetamide | ^{XX} | ^{XXX} | ≠ |
| | | | |
| (D,L)-2-Acetamido-N-benzyl-2-(methylamino)acetamide | 95.0 | 44.5 (37.0-52.4)* | ≠ |
| | | | |
| (D,L)-2-Acetamido-N-benzyl-2-(ethylamino)acetamide | X | 42.4 (37.2-47.8)* | ≠ |
| | | | |
| (D,L)-2-Acetamido-N-benzyl-3-indoleacetamide reference compound | x | ^{xxx} | ≠ |
| | | | |
| phenytoin | 66 | 10 | not effective |
| | | | |
| phenobarbital | 69 | 22 | 13 |
| | | | |
| mephenytoin | 154 | 61 | 31 |
| | | | |
| phenacemide | 421 (337-549)* | 87 (74-100)* | 116 (71-150)* |

| | | | |
|---|---|---|---|
| * 95% confidence intervals. | | | |
| ≠ The ED50 for this substrate was not computed. | | | |
| x The TD50 for this substrate was not computed. | | | |
| xx The TD50 value was determined using the horizontal screen test. | | | |
| XXX No activity was noted at ≤ 300 mg/kg | | | |

## Claims (Claims for the following Contracting State(s): AT, BE, CH, LI, DE, DK, FR, GB, IT, LU, NL, SE)

1. A compound of the formula: 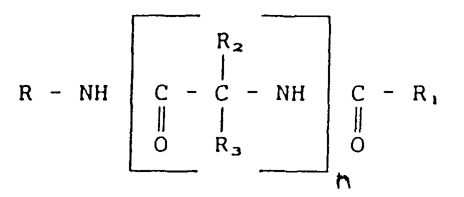
wherein R is aryl, aryl lower alkyl, heterocyclic or heterocyclic lower alkyl which R is unsubstituted or substituted with at least one electron withdrawing group or at least one electron donating group;
R₁ is hydrogen or lower alkyl which is unsubstituted or substituted with at least one electron withdrawing group or one electron donating group;
R₂ and R₃ are independently hydrogen or Z-Y which may be unsubstituted or substituted with at least one electron withdrawing group or one electron donating group, with the proviso that R₂ and R₃ cannot both be hydrogen;
Z is 0, S, NR₄ or PR₄;
Y is aryl lower alkyl, lower alkenyl or lower alkynyl, and Y may be unsubstituted or substituted with an electron donating group or an electron withdrawing group; or
ZY taken together is NR₄NR₅R₆, NR₄OR₅, OPR₄R₅, PR₄OR₅, SNR₄R₅, NR₄R₅, NR₄SR₅, SPR₄R₅, PR₄SR₅, NR₄PR₅R₆ or PR₄NR₅R₆;
R₄, R₅ and R₆ are independently hydrogen, lower alkyl, aryl, aryl lower alkyl, lower alkenyl, or lower alkynyl, wherein R₄, R₅ and R₆ may be unsubstituted or substituted with an electron withdrawing group or an electron donating group; and n is 1-4; or wherein
R is benzyl
R₁ is methyl
R₂ is hydrogen
R₃ is 2-(5-methylfuryl), 2-benzofuryl, 2-benzo[b]-thienyl, 2(5-methylpyrrolyl), or 2-pyridyl; and
n is 1; or
wherein
R is 2-fluorobenzyl, 3-fluorobenzyl, 4-fluorobenzyl, 2,5-difluorobenzyl, or 2,6-difluorobenzyl,
R₁ is methyl,
R₂ is hydrogen
R₃ is furyl, and
n is 1,
and the pharmaceutical acceptable salts thereof.

2. The compound according to Claim 1 wherein n is 1.

3. The compound according to Claim 1 or 2 wherein R₁ is methyl.

4. The compound according to any of Claims 1-3 wherein R is aryl lower alkyl.

5. The compound according to any.of Claims 1-4 wherein R is benzyl which is either unsubstituted or substituted with fluoro.

6. The compound according to any of Claims 1-5 wherein the electron withdrawing group is halo, nitro, lower alkanoyl, aryloyl, aryl lower alkanoyl, carboxy, carbalkoxy, carboxamido, cyano, sulfonyl, sulfoxide, heterocyclic, guanidine or quaternary ammonium.

7. The compound according to any of Claims 1-5 wherein the electron donating group is hydroxy, lower alkoxy, lower alkyl, amino, lower alkylamino, di(loweralkyl)amino, phenoxy, thiol, lower alkylmercapto or disulfide.

8. The compound according to any of Claims 1-7 wherein one of the R₂ and R₃ is hydrogen.

9. The compound according to any of Claims 1-8 wherein one of the R₂ and R₃ is hydrogen and the other is Z-Y.

10. The compound according to any of Claims 1-9 wherein Z-Y is hydrazino, lower alkylhydrazino, N-phenylhydrazino, N-hydroxylanino, or O-hydroxylamino.

11. The compound according to an of Claims 1-5 which is
(D,L)-α-Acetamido-N-benzyl-2-(5-methylfuran)acetamide,
(D,L)-α-Acetamido-N-benzyl-2-benzofuranacetamide,
(D,L)-α-Acetamido-N-benzyl-2-benzo[b]-thiopheneacetamide,
(D,L)-α-Acetamido-N-benzyl-2-(5-methylpyrrole)acetamide,
(D,L)-α-Acetamido-N-(2-fluorobenzyl)-2-furanacetamide,
(D,L)-α-Acetamido-N-(3-fluorobenzyl)-2-furanacetamide,
(D,L)-α-Acetamido-N-(4-fluorobenzyl)-2-furanacetamide,
(D,L)-α-Acetamido-N-(2,5-difluorobenzyl)-2-furanacetamide,
(D,L)-α-Acetamido-N-(2,6-difluorobenzyl)-2-furanacetamide,
(D,L)-2-Acetamido-4-pentenoic acid-N-benzylamide,
(D,L)-2-Acetamido-N-benzyl-2-(N-anilino)acetamide,
(D,L)-2-Acetamido-N-benzyl-2-(methylamino)acetamide, or
(D,L)-2-Acetamido-N-benzyl-2-(ethylamino)acetamide,
or the D or L stereoisomer thereof.

12. The compound according to Claim 11 wherein the compound is the D-stereoisomer.

13. An anticonvulsant composition comprising an anticonvulsant effective amount of a compound according to any one of Claims 1-12 and a pharmaceutically acceptable carrier therefor.

14. The composition of Claim 3 having a unit dosage form containing from 5 to 1000 mg of said compound.

15. A process for the preparation of a compound of the formula I 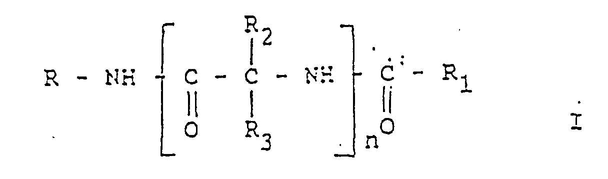 as in claim 1 which comprises
a.) reacting an amine of Formula II 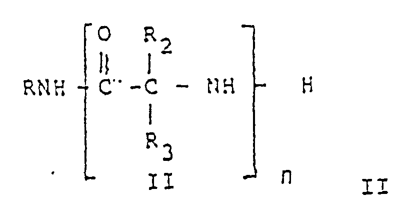 wherein R, R₂ an R₃ have the same meaning as in formula I with an acylating derivative of a compound of formula III 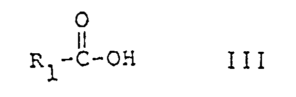 wherein R₁ has the same meaning as in claim 1 under amide forming conditions; or
b.) reacting a compound of formula VI 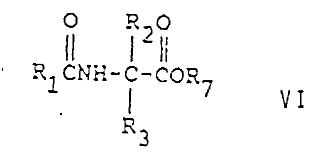 wherein R₁, R₂ and R₃ have the same meaning as in claim 1 and R₇ is lower alkyl, aryl or aryl lower alkyl or an acylating derivative thereof with an amine of formula RNH₂ wherein R has the same meaning as in claim 1 under amide forming conditions; or
c.) reacting an amide of formula IX 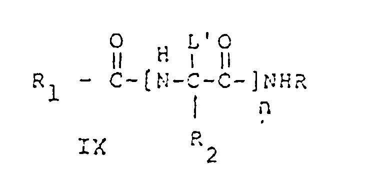
wherein R, R₁, R₂ and n have the same meaning as in claim 1 with an acylating derivative of formula X
R₃-L X
wherein R₃ has the same meaning as in claim 1 but is not aryl, hetero-aromatic or polynuclear aromatic and L and L' are independently a good leaving group, such as halide, tosylates, mesoylates, brosylates and benzyloxy under substitution conditions;
and optionally removing protecting groups when present; and optionally introducing substituents into the compounds by substitution or conversion reactions; and optionally separating out the diastereomers, or optionally forming pharmaceutically acceptable salts of said compounds.

16. The process according to Claim 15 wherein n is 1.

17. The process according to Claim 15 or 16, wherein R₁ is methyl.

18. The process according to any of Claims 15-17 wherein R is aryl lower alkyl.

19. The process according to any of Claims 15-18 wherein R is benzyl which is either unsubstituted or substituted with fluoro.

20. The process according to any of Claims 15-19, wherein the electron withdrawing group is halo, nitro, lower alkanoyl, aryloyl, aryl lower alkanoyl, carboxy, carbalkoxy, carboxamido, cyano, sulfonyl, sulfoxide, heterocyclic, guanidine or quaternary ammonium.

21. The process according to any of Claims 15-19 wherein the electron donating group is hydroxy, lower alkoxy, lower alkyl, amino, lower alkylamino, di(loweralkyl)amino, phenoxy, thiol, lower alkylmercapto or disulfide.

22. The process according to any of Claims 15-21 wherein one of the R₂ and R₃ is hydrogen.

23. The process according to any of Claims 15-22 wherein one of the R₂ and R₃ is hydrogen and the other is Z-Y.

24. The process according to any of Claims 15-22 wherein Z-Y is hydrazino, lower alkylhydrazino, N-phenylhydrazino, N-hydroxylamino, or O-hydroxylamino.

25. The process according to any of Claims 15-19 wherein the compound formed is selected from
(D,L)-α-Acetamido-N-benzyl-2-(5-methylfuran)acetamide,
(D,L)-α-Acetamido-N-benzyl-2-benzofuranacetamide,
(D,L)-α-Acetamido-N-benzyl-2-benzo[b]-thiopheneacetamide,
(D,L)-α-Acetamido-N-benzyl-2-(5-methylpyrrole)acetamide,
(D,L)-α-Acetamido-N-(2-fluorobenzyl)-2-furanacetamide,
(D,L)-α-Acetamido-N-(3-fluorobenzyl)-2-furanacetamide,
(D,L)-α-Acetamido-N-(4-fluorobenzyl)-2-furanacetamide,
(D,L)-α-Acetamido-N-(2,5-difluorobenzyl)-2-furanacetamide,
(D,L)-α-Acetamido-N-(2,6-difluorobenzyl)-2-furanacetamide,
(D,L)-2-Acetamido-4-pentenoic acid-N-benzylamide,
(D,L)-2-Acetamido-N-benzyl-2-(N-anilino)acetamide,
(D,L)-2-Acetamido-N-benzyl-2-(methylamino)acetamide, and
(D,L)-2-Acetamido-N-benzyl-2-(ethylamino)acetamide,
or the D or L stereoisomer thereof.

26. The process according to Claim 25 wherein the compound formed is the D-stereoisomer.

27. The process for the preparation of an enantiomer of Acetamido-N-benzyl-2-furanacetamide which comprises reacting racemic α-acetamido-2-furanacetic acid or acylating derivative thereof with one of the stereoisomers of an optically active amine having one chiral center under conditions effective to form the corresponding diasteromeric salt, separating the diasteromeric salts to obtain the desired salt, hydrolyzing the salt under conditions effective to form the desired α-acetamido-2-furanacetic acid and reacting the product thus formed with benzylamine under amide forming conditions.

28. The process according to Claim 27 wherein the racemic α-acetamide-2-furanacetic acid is prepared by reacting furan with an acylating derivative of acetamido-2-haloacetic acid wherein the halo group is bromo or chloro.

29. Use of compounds according to the preceding claims for preparing a medicament for treatment of epilepsy and other CNS disorders.

## Claims (Claims for the following Contracting State(s): ES, GR)

1. A process for the preparation of compound of the formula: 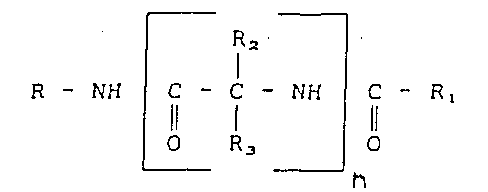
wherein R is aryl, aryl lower alkyl, heterocyclic or heterocyclic lower alkyl which R is unsubstituted or substituted with at least one electron withdrawing group or at least one electron donating group;
R₁ is hydrogen or lower alkyl which is unsubstituted or substituted with at least one electron withdrawing group or one electron donating group;
R₂ and R₃ are independently hydrogen or Z-Y which may be unsubstituted or substituted with at least one electron withdrawing group or one electron donating group, with the proviso that R₂ and R₃ cannot both be hydrogen;
Z is 0, S, NR₄ or PR₄;
Y is aryl lower alkyl, lower alkenyl or lower alkynyl, and Y may be unsubstituted or substituted with an electron donating group or an electron withdrawing group; or
ZY taken together is NR₄NR₅R₆, NR₄OR₅, OPR₄R₅, PR₄OR₅, SNR₄R₅, NR₄R₅, NR₄SR₅, SPR₄R₅, PR₄SR₅, NR₄PR₅R₆ or PR₄NR₅R₆;
R₄, R₅ and R₆ are independently hydrogen, lower alkyl, aryl, aryl lower alkyl, lower alkenyl, or lower alkynyl, wherein R₄, R₅ and R₆ may be unsubstituted or substituted with an electron withdrawing group or an electron, donating group; and n is 1-4; or wherein
R is benzyl
R₁ is methyl
R₂ is hydrogen
R₃ is 2-(5-methylfuryl), 2-benzofuryl, 2-benzo[b]-thienyl, 2(5-methylpyrrolyl), or 2-pyridyl; and
n is 1; or
wherein
R is 2-fluorobenzyl, 3-fluorobenzyl, 4-fluorobenzyl, 2,5-difluorobenzyl, or 2,6-difluorobenzyl,
R₁ is methyl,
R₂ is hydrogen
R₃ is furyl, and
n is 1,
and the pharmaceutical acceptable salts thereof, comprising
a.) reacting an amine of Formula II 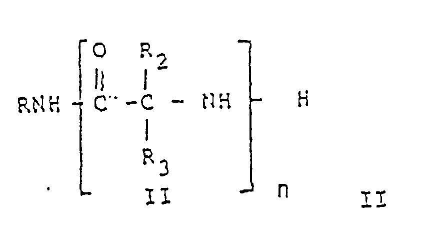 wherein R, R₂ an R₃ have the same meaning as in formula I with an acylating derivative of a compound of formula III 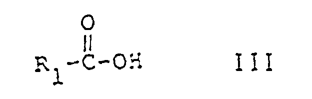 wherein R₁ has the same meaning as above under amide forming conditions; or
b.) reacting a compound or formula VI 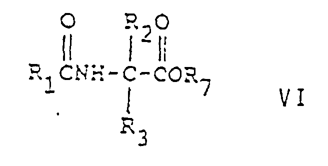 wherein R₁, R₂ and R₃ have the same meaning as above and R₇ is lower alkyl, aryl or aryl lower alkyl or an acylating derivative thereof with an amine of formula RNH₂ wherein R has the same meaning as above under amide forming conditions; or
c.) reacting an amide of formula IX 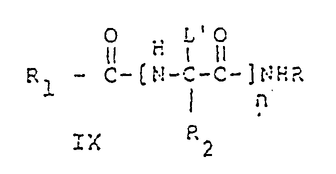
wherein R, R₁, R₂ and n have the same meaning as above with an acylating derivative of formula X
R₃-L X
wherein R₃ has the same meaning as above but is not aryl, hetero-aromatic or polynuclear aromatic and L and L' are independently a good leaving group, such as halide, tosylates, mesoylates, brosylates and benzyloxy under substitution conditions;
and optionally removing protecting groups when present; and optionally introducing substituents into the compounds by substitution or conversion reactions; and optionally separating out the diastereomers, or optionally forming pharmaceutically acceptable salts of said compounds.

2. The process according to Claim 1 wherein n is 1.

3. The process according to Claim 1 or 2, wherein R₁ is methyl.

4. The process according to any of Claims 1-3 wherein R is aryl lower alkyl.

5. The process according to any of Claims 1-4 wherein R is benzyl which is either unsubstituted or substituted with fluoro.

6. The process according to any of Claims 1-5 wherein the electron withdrawing group is halo, nitro, lower alkanoyl, aryloyl, aryl lower alkanoyl, carboxy, carbalkoxy, carboxamido, cyano, sulfonyl, sulfoxide, heterocyclic, guanidine or quaternary ammonium.

7. The process according to any of Claims 1-5 Wherein the electron donating group is hydroxy, lower alkoxy, lower alkyl, amino, lower alkylamino, di(loweralkyl)amino, phenoxy, thiol, lower alkylmercapto or disulfide.

8. The process according to any of Claims 1-7 wherein one of the R₂ and R₃ is hydrogen.

9. The process according to any of Claims 1-8 wherein one of the R₂ and R₃ is hydrogen and the other is Z-Y.

10. The process according to any of Claims 1-9 wherein Z-Y is hydrazino, lower alkylhydrazino, N-phenylhydrazino, N-hydroxylamino, or O-hydroxylamino.

11. The process according to any of Claims 1-5 wherein the compound formed is selected from
(D,L)-α-Acetamido-N-benzyl-2-(5-methylfuran)acetamide,
(D,L)-α-Acetamido-N-benzyl-2-benzofuranacetamide,
(D,L)-α-Acetamido-N-benzyl-2-benzo[b]-thiopheneacetamide,
(D,L)-α-Acetamido-N-benzyl-2-(5-methylpyrrole)acetamide,
(D,L)-α-Acetamido-N-(2-fluorobenzyl)-2-furanacetamide,
(D,L)-α-Acetamido-N-(3-fluorobenzyl)-2-furanacetamide,
(D,L)-α-Acetamido-N-(4-fluorobenzyl)-2-furanacetamide,
(D,L)-α-Acetamido-N-(2,5-difluorobenzyl)-2-furanacetamide,
(D,L)-α-Acetamido-N-(2,6-difluorobenzyl)-2-furanacetamide,
(D,L)-2-Acetamido-4-pentenoic acid-N-benzylamide,
(D,L)-2-Acetamido-N-benzyl-2-(N-anilino)acetamide,
(D,L)-2-Acetamido-N-benzyl-2-(methylamino)acetamide, and
(D,L)-2-Acetamido-N-benzyl-2-(ethylamino)acetamide,
or the D or L stereoisomer thereof.

12. The process according to Claim 11 wherein the compound formed is the D-stereoisomer.

13. The process for the preparation of an enantiomer of Acetamido-N-benzyl-2-furanacetamide which comprises reacting racemic α-acetamido-2-furanacetic acid or acylating derivative thereof with one of the stereoisomers of an optically active amine having one chiral center under conditions effective to form the corresponding diasteromeric salt, separating the diasteromeric salts to obtain the desired salt, hydrolyzing the salt under conditions effective to form the desired α-acetamido-2-furanacetic acid and reacting the product thus formed with benzylamine under amide forming conditions.

14. The process according to claim 13 wherein the racemic α-acetamide-2-furanacetic acid is prepared by reacting furan with an acylating derivative of acetamido-2-haloacetic acid wherein the halo group is bromo or chloro.

15. Process for the preparation of a medicament for treatment of epilepsy and other CNS disorders using a compound of the formula I as defined in any of claims 1-12.

16. Use of a compound of the formula I as defined in any of claims 1 to 12 for preparing a medicament for treatment of epilepsy and other CNS disorders.

## Patentansprüche (Patentansprüche für folgende(n) Vertragsstaat(en): AT, BE, CH, LI, DE, DK, FR, GB, IT, LU, NL, SE)

1. Verbindung der Formel: 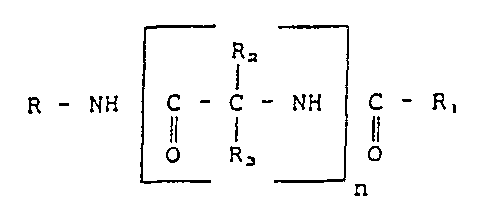
worin R Aryl, Arylniedrigalkyl, Hetereocyclus, heterocyclisches Niedrigalkyl ist, wobei R unsubstituiert ist oder mit mindestens einer elektronenanziehenden Gruppe oder mindestens einer elektronenabgebenden Gruppe substituiert ist;
R₁ Wasserstoff oder Niedrigalkyl, das unsubstituiert ist oder mit mindestens einer elektronenanziehenden oder einer elektronenabgebenden Gruppe substituiert ist, ist;
R₂ und R₃ unabhängig voneinander Wasserstoff oder Z-Y, das unsubstituiert sein kann oder mit mindestens einer elektronenanziehenden Gruppe oder mindestens einer elektronenabgebenden Gruppe substituiert sein kann, sind, mit der Maßgabe, daß R₂ und R₃ nicht beide Wasserstoff sein können;
Z O, S NR₄ oder PR₄ ist;
Y Arylniedrigalkyl, Niedrigalkenyl oder Niedrigalkinyl ist und Y unsubstituiert oder mit einer elektronenabgebenden Gruppe oder einer elektronenaziehenden Gruppe substituiert sein kann; oder
ZY zusammengenommen NR₄NR₅R₆, NR₄OR₅, OPR₄R₅, PR₄OR₅, SNR₄R₅, NR₄R₅, NR₄SR₅, SPR₄R₅, PR₄SR₅, NR₄PR₅R₆ oder PR₄NR₅R₆ ist;
R₄, R₅ und R₆ unabhängig voneinander Wasserstoff, Niedrigalkyl, Aryl, Arylniedrigalkyl, Niedrigalkenyl oder Niedrigalkinyl sind, wobei R₄, R₅ und R₆ unsubstituiert oder mit einer elektronenanziehenden oder einer elektronenabgebenden Gruppe substituiert sein können; und n 1 bis 4 ist, oder
worin
R Benzyl ist,
R₁ Methyl ist,
R₂ Wasserstoff ist,
R₃ 2-(5-Methylfuryl), 2-Benzofuryl, 2-Benzo[b]-thienyl, 2(5-Methylpyrrolyl) oder 2-Pyridyl ist; oder
worin
R 2-Fluorbenzyl, 3-Fluorbenzyl, 4-Fluorbenzyl,
2,5-Difluorbenzyl oder 2,6-Difluorbenzyl ist;
R₁ Methyl ist,
R₂ Wasserstoff ist,
R₃ Furyl ist und
n 1 ist;
und die pharmazeutisch annehmbaren Salze derselben.

2. Verbindung nach Anspruch 1, wobei n 1 ist.

3. Verbindung nach Anspruch 1 oder Anspruch 2, wobei R₁ Methyl ist.

4. Verbindung nach einem der Ansprüche 1 bis 3, wobei R Arylniedrigalkyl ist.

5. Verbindung nach einem der Ansprüche 1 bis 4, wobei R Benzyl ist, das entweder unsubstituiert ist oder mit Fluor substituiert ist.

6. Verbindung nach einem der Ansprüche 1 bis 5, wobei die elektronenanziehende Gruppe Halogen, Nitro, Niedrigalkanoyl, Aryloyl, Arylniedrigalkanoyl, Carboxy, Carbalkoxy, Carboxamido, Cyano, Sulfonyl, Sulfoxid, Heterocyclus, Guanidin oder quaternäres Ammonium ist.

7. Verbindung nach einem der Ansprüche 1 bis 5, wobei die elektronenabgebende Gruppe Hydroxy, Niedrigalkoxy, Niedrigalkyl, Amino, Niedrigalkylamino, Di(niedrigalkyl)amino, Phenoxy, Thiol, Niedrigalkylmercapto oder Disulfid ist.

8. Verbindung nach einem der Ansprüche 1 bis 7, wobei einer der Reste R₂ und R₃ Wasserstoff ist.

9. Verbindung nach einem der Ansprüche 1 bis 8, wobei einer der Reste R₂ und R₃ Wasserstoff ist und der andere Z-Y ist.

10. Verbindung nach einem der Ansprüche 1 bis 9, wobei Z-Y Hydrazino, Niedrigalkylhydrazino, N-Phenylhydrazino, N-Hydroxylamino oder O-Hydroxylamino ist.

11. Verbindung nach einem der Ansprüche 1 bis 5, die
(D,L)-α-Acetamido-N-benzyl-2-(5-methylfuran)acetamid,
(D,L)-α-Acetamido-N-benzyl-2-benzofuranacetamid,
(D,L)-α-Acetamido-N-benzyl-2-benzo[b]-thiophenacetamid,
(D,L)-α-Acetamido-N-benzyl-2-(5-methylpyrrol)acetamid,
(D,L)-α-Acetamido-N-(2-fluorbenzyl)-2-furanacetamid,
(D,L)-α-Acetamido-N-(3-fluorbenzyl)-2-furanacetamid,
(D,L)-α-Acetamido-N-(4-fluorbenzyl)-2-furanacetamid,
(D,L)-α-Acetamido-N-(2,5-difluorbenzyl)-2-furanacetamid,
(D,L)-2-Acetamido-N-(2,6-difluorbenzyl)-2-furanacetamid,
(D,L)-2-Acetamido-4-pentensäure-N-benzylamid,
(D,L)-2-Acetamido-N-benzyl-2-(N-anilino)acetamid,
(D,L)-2-Acetamido-N-benzyl-2-(methylamino)acetamid oder
(D,L)-2-Acetamido-N-benzyl-2-(ethylamino)acetamid oder das D- oder L- Stereoisomer davon ist.

12. Verbindung nach Anspruch 11, wobei die Verbindung das D-Stereoisomer ist.

13. Antikonvulsivum-Zusammensetzung, die eine als Δntikonvulsivum wirksame Menge einer Verbindung nach einem der Ansprüche 1 bis 12 und einen pharmazeutisch annehmbaren Träger dafür umfaßt.

14. Zusammensetzung nach Anspruch 13, die eine Einheitsdosierungsform hat, die 5 bis 1000 mg der Verbindung enthält.

15. Verfahren zur Herstellung einer Verbindung der Formel (I) nach Anspruch 1: 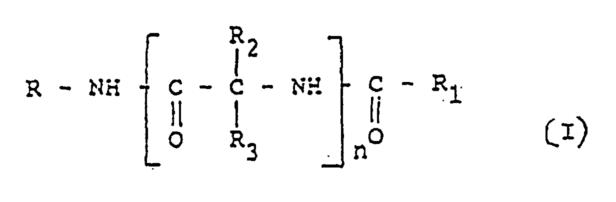 umfassend
a) Umsetzen eines Amins der Formel (II): 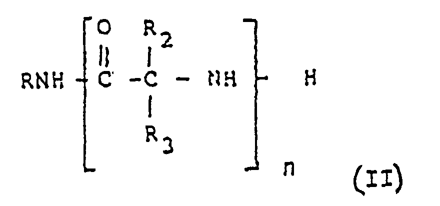 worin R, R₂ and R₃ dieselbe Bedeutung wie bei Formel (I) haben, mit einem Acylierungs-Derivat einer Verbindung der Formel (III): 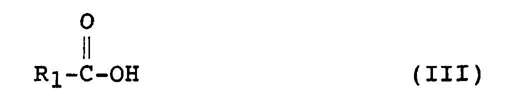 worin R₁ dieselbe Bedeutung wie in Anspruch 1 hat, unter Amid-Bildungsbedingungen; oder
b) Umsetzen einer Verbindung der Formel (VI): 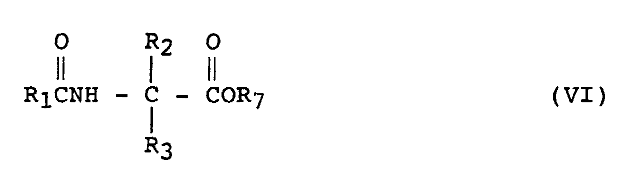 worin R₁, R₂ und R₃ dieselbe Bedeutung wie in Anspruch 1 haben und R₇ Niedrigalkyl, Aryl oder Arylniedrigalkyl ist, oder eines Acylierungs-Derivats davon mit einem Amin der Formel RNH₂, worin R dieselbe Bedeutung wie in Anspruch 1 hat, unter Amid-Bildungsbedingungen; oder
c) Umsetzen eines Amids der Formel (IX): 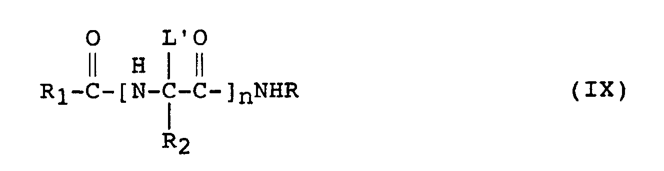 worin R, R₁, R₂ and n dieselbe Bedeutung wie in Anspruch 1 haben, mit einem Acylierungs-Derivat der Formel (X):
R₃-L (X)
worin R₃ dieselbe Bedeutung wie in Anspruch 1 hat, aber kein Aryl, Heteroaromat oder mehrkerniger Aromat ist und L und L' unabhängig voneinander ein gute Abgangsgruppe wie z.B. Halogenid, Tosylate, Mesoylate, Brosylate und Benzyloxy sind, unter Substitutionsbedingungen;
und gegebenenfalls Entfernen von Schutzgruppen, wenn die vorhanden sind; und gegebenenfalls Einführen von Substituenten in die Verbindungen durch Substitutions- oder Umwandlungsreaktionen; und gegebenenfalls Trennen der Diastereomeren oder gegebenenfalls Bilden von pharmazeutisch annehmbaren Salzen dieser Verbindungen.

16. Verfahren nach Anspruch 15, wobei n 1 ist.

17. Verfahren nach Anspruch 15 oder 16, wobei R₁ Methyl ist.

18. Verfahren nach einem der Ansprüche 15 bis 17, wobei R Arylniedrigalkyl ist.

19. Verfahren nach einem der Ansprüche 15 bis 18, wobei R Benzyl ist, das entweder unsubstituiert oder mit Fluor substituiert ist.

20. Verfahren nach einem der Ansprüche 15 bis 19, wobei die elektronenanziehende Gruppe Halogen, Nitro, Niedrigalkanoyl, Aryloyl, Arylniedrigalkanoyl, Carboxy, Carbalkoxy, Carboxamido, Cyano, Sulfonyl, Sulfoxid, Heterocyclus, Guanidin oder quaternäres Ammonium ist.

21. Verfahren nach einem der Ansprüche 15 bis 19, wobei die elektronenabgebende Gruppe Hydroxy, Niedrigalkoxy, Niedrigalkyl, Amino, Niedrigalkylamino, Di(niedrigalkyl)amino, Phenoxy, Thiol, Niedrigalkylmercapto oder Disulfid ist.

22. Verfahren nach einem der Ansprüche 15 bis 21, wobei einer der Reste R₂ und R₃ Wasserstoff ist.

23. Verfahren nach einem der Ansprüche 15 bis 22, wobei einer der Reste R₂ und R₃ Wasserstoff ist und der andere Z-Y ist.

24. Verfahren nach einem der Ansprüche 15 bis 22, wobei Z-Y Hydrazino, Niedrigalkylhydrazino, N-Phenylhydrazino, N-Hydroxylamino oder O-Hydroxylamino ist.

25. Verfahren nach einem der Ansprüche 15 bis 19, wobei die Verbindung ausgewählt ist aus:
(D,L)-α-Acetamido-N-benzyl-2-(5-methylfuran)acetamid,
(D,L)-α-Acetamido-N-benzyl-2-benzofuranacetamid,
(D,L)-α-Acetamido-N-benzyl-2-benzo[b]-thiophenacetamid,
(D,L)-α-Acetamido-N-benzyl-2-(5-methylpyrrol)acetamid,
(D,L)-α-Acetamido-N-(2-fluorbenzyl)-2-furanacetamid,
(D,L)-α-Acetamido-N-(3-fluorbenzyl)-2-furanacetamid,
(D,L)-α-Acetamido-N-(4-fluorbenzyl)-2-furanacetamid,
(D,L)-α-Acetamido-N-(2,5-difluorbenzyl)-2-furanacetamid,
(D,L)-2-Acetamido-N-(2,6-difluorbenzyl)-2-furanacetamid,
(D,L)-2-Acetamido-4-pentensäure-N-benzylamid,
(D,L)-2-Acetamido-N-benzyl-2-(N-anilino)acetamid,
(D,L)-2-Acetamido-N-benzyl-2-(methylamino)acetamid oder
(D,L)-2-Acetamido-N-benzyl-2-(ethylamino)acetamid und dem D- und L- Stereoisomer davon.

26. Verfahren nach Anspruch 25, wobei die gebildete Verbindung das D-Stereoisomer ist.

27. Verfahren zur Herstellung eine Enantiomeren von Acetamido-N-benzyl-2-furanacetamid, das Umsetzen von racemischer α-Acetamido-2-furanessigsäure oder einem Acylierungs-Derivat davon mit einem der Stereoisomeren eines optisch aktiven Amins, das ein chirales Zentrum hat, unter Bedingungen, die zu Bildung des entsprechenden diastereomeren Salzes wirksam sind, Trennen der diastereomeren Salze unter Erhalt des gewünschten Salzes, Hydrolysieren des Salzes unter Bedingungen, die zur Bildung der gewünschten α-Acetamido-2-furanessigsäure wirksam sind, und Umsetzen des so gebildeten Produktes mit Benzylamin unter Amid-Bildungsbedingungen umfaßt.

28. Verfahren nach Anspruch 27, wobei die racemische α-Acetamido-2-furanessigsäure durch Umsetzen von Furan mit einem Acylierungs-Derivat von 2-Acetamido-2-halogenessigsäure, in der die Halogen-Gruppe Brom oder Chlor ist, hergestellt wird.

29. Verwendung von Verbindungen nach den vorangehenden Ansprüchen zur Herstellung eines Arzneimittels zur Behandlung von Epilepsie und anderen ZNS-Erkrankungen.

## Patentansprüche (Patentansprüche für folgende(n) Vertragsstaat(en): ES, GR)

1. Verfahren zur Herstellung einer Verbindung der Formel: 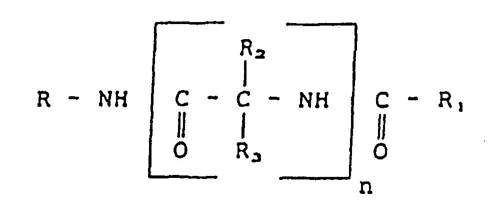
worin R Aryl, Arylniedrigalkyl, Hetereocyclus, heterocyclisches Niedrigalkyl ist, wobei R unsubstituiert ist oder mit mindestens einer elektronenanziehenden Gruppe oder mindestens einer elektronenabgebenden Gruppe substituiert ist;
R₁ Wasserstoff oder Niedrigalkyl, das unsubstituiert ist oder mit mindestens einer elektronenanziehenden oder einer elektronenabgebenden Gruppe substituiert ist, ist;
R₂ und R₃ unabhängig voneinander Wasserstoff oder Z-Y, das unsubstituiert sein kann oder mit mindestens einer elektronenanziehenden Gruppe oder mindestens einer elektronenabgebenden Gruppe substituiert sein kann, sind, mit der Maßgabe, daß R₂ und R₃ nicht beide Wasserstoff sein können;
Z O, S NR₄ oder PR₄ ist;
Y Arylniedrigalkyl, Niedrigalkenyl oder Niedrigalkinyl ist und Y unsubstituiert oder mit einer elektronenabgebenden Gruppe oder einer elektronenanziehenden Gruppe substituiert sein kann; oder
ZY zusammengenommen NR₄NR₅R₆, NR₄OR₅, OPR₄R₅, PR₄OR₅, SNR₄R₅, NR₄R₅, NR₄SR₅, SPR₄R₅, PR₄SR₅, NR₄PR₅R₆ oder PR₄NR₅R₆ ist;
R₄, R₅ und R₆ unabhängig voneinander Wasserstoff, Niedrigalkyl, Aryl, Arylniedrigalkyl, Niedrigalkenyl oder Niedrigalkinyl sind, wobei R₄, R₅ und R₆ unsubstituiert oder mit einer elektronenanziehenden oder einer elektronenabgebenden Gruppe substituiert sein können; und n 1 bis 4 ist, oder
worin
R Benzyl ist,
R₁ Methyl ist,
R₂ Wasserstoff ist,
R₃ 2-(5-Methylfuryl), 2-Benzofuryl, 2-Benzo[b]-thienyl, 2(5-Methylpyrrolyl) oder 2-Pyridyl ist; oder
worin
R 2-Fluorbenzyl, 3-Fluorbenzyl, 4-Fluorbenzyl,
2,5-Difluorbenzyl oder 2,6-Difluorbenzyl ist;
R₁ Methyl ist,
R₂ Wasserstoff ist,
R₃ Furyl ist und
n 1 ist;
und der pharmazeutisch annehmbaren Salze derselben,
umfassend:
a) Umsetzen eines Amins der Formel (II): 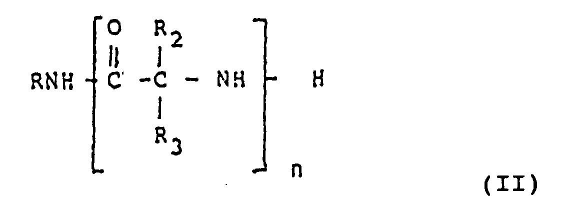 worin R, R₂ und R₃ dieselbe Bedeutung wie bei Formel (I) haben, mit einem Acylierungs-Derivat einer Verbindung der Formel (III): 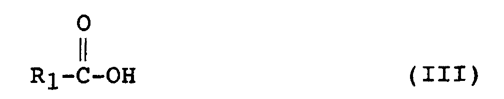 worin R₁ dieselbe Bedeutung wie in Anspruch 1 hat, unter Amid-Bildungsbedingungen; oder
b) Umsetzen einer Verbindung der Formel (VI): 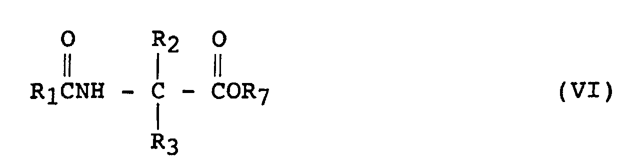 worin R₁, R₂ und R₃ dieselbe Bedeutung wie in Anspruch 1 haben und R₇ Niedrigalkyl, Aryl oder Arylniedrigalkyl ist, oder eines Acylierungs-Derivats davon mit einem Amin der Formel RNH₂, worin R dieselbe Bedeutung wie in Anspruch 1 hat, unter Amid-Bildungsbedingungen; oder
c) Umsetzen eines Amids der Formel (IX): 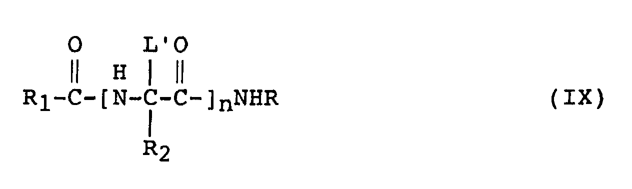 worin R, R₁, R₂ und n dieselbe Bedeutung wie in Anspruch 1 haben, mit einem Acylierungs-Derivat der Formel (X):
R₃-L (X)
worin R₃ dieselbe Bedeutung wie in Anspruch 1 hat, aber kein Aryl, Heteroaromat oder mehrkerniger Aromat ist und L und L' unabhängig voneinander ein gute Abgangsgruppe wie z.B. Halogenid, Tosylate, Mesoylate, Brosylate und Benzyloxy sind, unter Substitutionsbedingungen;
und gegebenenfalls Entfernen von Schutzgruppen, wenn die vorhanden sind; und gegebenenfalls Einführen von Substituenten in die Verbindungen durch Substitutions- oder Umwandlungsreaktionen; und gegebenenfalls Trennen der Diastereomeren oder gegebenenfalls Bilden von pharmazeutisch annehmbaren Salzen dieser Verbindungen.

2. Verfahren nach Anspruch 1, wobei n 1 ist.

3. Verfahren nach Anspruch 1 oder Anspruch 2, wobei R₁ Methyl ist.

4. Verfahren nach einem der Ansprüche 1 bis 3, wobei R Arylniedrigalkyl ist.

5. Verfahren nach einem der Ansprüche 1 bis 4, wobei R Benzyl ist, das entweder unsubstituiert ist oder mit Fluor substituiert ist.

6. Verfahren nach einem der Ansprüche 1 bis 5, wobei die elektronenanziehende Gruppe Halogen, Nitro, Niedrigalkanoyl, Aryloyl, Arylniedrigalkanoyl, Carboxy, Carbalkoxy, Carboxamido, Cyano, Sulfonyl, Sulfoxid, Heterocyclus, Guanidin oder quaternäres Ammonium ist.

7. Verfahren nach einem der Ansprüche 1 bis 5, wobei die elektronenabgebende Gruppe Hydroxy, Niedrigalkoxy, Niedrigalkyl, Amino, Niedrigalkylamino, Di(niedrigalkyl)amino, Phenoxy, Thiol, Niedrigalkylmercapto oder Disulfid ist.

8. Verfahren nach einem der Ansprüche 1 bis 7, wobei einer der Reste R₂ und R₃ Wasserstoff ist.

9. Verfahren nach einem der Ansprüche 1 bis 8, wobei einer der Reste R₂ und R₃ Wasserstoff ist und der andere Z-Y ist.

10. Verfahren nach einem der Ansprüche 1 bis 9, wobei Z-Y Hydrazino, Niedrigalkylhydrazino, N-Phenylhydrazino, N-Hydroxylamino oder O-Hydroxylamino ist.

11. Verfahren nach einem der Ansprüche 1 bis 5, wobei die Verbindung ausgewählt ist aus:
(D,L)-α-Acetamido-N-benzyl-2-(5-methylfuran)acetamid,
(D,L)-α-Acetamido-N-benzyl-2-benzofuranacetamid,
(D,L)-α-Acetamido-N-benzyl-2-benzo[b]-thiophenacetamid,
(D,L)-α-Acetamido-N-benzyl-2-(5-methylpyrrol)acetamid,
(D,L)-α-Acetamido-N-(2-fluorbenzyl)-2-furanacetamid,
(D,L)-α-Acetamido-N-(3-fluorbenzyl)-2-furanacetamid,
(D,L)-α-Acetamido-N-(4-fluorbenzyl)-2-furanacetamid,
(D,L)-α-Acetamido-N-(2,5-difluorbenzyl)-2-furanacetamid,
(D,L)-2-Acetamido-N-(2,6-difluorbenzyl)-2-furanacetamid,
(D,L)-2-Acetamido-4-pentensäure-N-benzylamid,
(D,L)-2-Acetamido-N-benzyl-2-(N-anilino)acetamid,
(D,L)-2-Acetamido-N-benzyl-2-(methylamino)acetamid oder
(D,L)-2-Acetamido-N-benzyl-2-(ethylamino)acetamid und dem D- und L- Stereoisomer davon.

12. Verfahren nach Anspruch 11, wobei die gebildete Verbindung das D-Stereoisomer ist.

13. Verfahren zur Herstellung eine Enantiomeren von Acetamido-N-benzyl-2-furanacetamid, das Umsetzen von racemischer α-Acetamido-2-furanessigsäure oder einem Acylierungs-Derivat davon mit einem der Stereoisomeren eines optisch aktiven Amins, das ein chirales Zentrum hat, unter Bedingungen, die zu Bildung des entsprechenden diastereomeren Salzes wirksam sind, Trennen der diastereomeren Salze unter Erhalt des gewünschten Salzes, Hydrolysieren des Salzes unter Bedingungen, die zur Bildung der gewünschten α-Acetamido-2-furanessigsäure wirksam sind, und Umsetzen des so gebildeten Produktes mit Benzylamin unter Amid-Bildungsbedingungen umfaßt.

14. Verfahren nach Anspruch 13, wobei die racemische α-Acetamido-2-furanessigsäure durch umsetzen von Furan mit einem Acylierungs-Derivat von 2-Acetamido-2-halogenessigsäure, in der die Halogen-Gruppe Brom oder Chlor ist, hergestellt wird.

15. Verfahren zur Herstellung eines Arzneimittels zur Behandlung von Epilepsie und anderen ZNS-Erkrankungen unter Verwendung einer Verbindung der Formel (I), wie sie in einem der Ansprüche 1 bis 12 definiert ist.

16. Verwendung einer Verbindung der Formel (I), wie sie in einem der Ansprüche 1 bis 12 definiert ist, zur Herstellung eines Medikaments zur Behandlung von Epilepsie und anderen ZNS-Erkrankungen.

## Revendications (Revendications pour l'(les) Etat(s) contractant(s) suivant(s): AT, BE, CH, LI, DE, DK, FR, GB, IT, LU, NL, SE)

1. Composé de formule : 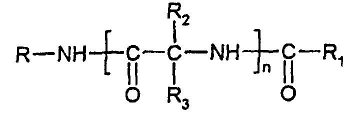 dans laquelle
R est un radical aryle, arylalkyle inférieur, hétérocyclique, hétérocycloalkyle inférieur, lequel R est non substitué ou est substitué par au moins un groupe extracteur d'électrons ou groupe donneur d'électrons ;
R₁ est l'hydrogène ou un radical alkyle inférieur, non substitué ou substitué par un groupe donneur d'électrons ou un groupe extracteur d'électrons ;
R₂ et R₃ sont indépendamment l'hydrogène ou Z-Y qui peut être non substitué ou substitué par au moins un groupe extracteur d'électrons ou un groupe donneur d'électrons, à condition que R₂ et R₃ ne peuvent pas être tous deux l'hydrogène ;
Z est O, S, NR₄ ou PR₄ ;
Y est un radical arylalkyle inférieur, alcényle inférieur ou alcynyle inférieur, et Y peut être non substitué ou substitué par un groupe donneur d'électrons ou un groupe extracteur d'électrons ; ou bien
ZY, pris ensemble, est NR₄NR₅R₆, NR₄OR₅, OPR₄R₅, PR₄OR₅, SNR₄R₅, NR₄R₅, NR₄SR₅, SPR₄R₅, PR₄SR₅, NR₄PR₅R₆ ou PR₄NR₅R₆ ;
R₄, R₅ et R₆ sont indépendamment l'hydrogène ou un radical alkyle inférieur, aryle, arylalkyle inférieur, alcényle inférieur ou alcynyle inférieur, où R₄, R₅ et R₆ peuvent être non substitués ou substitués par un groupe soustracteur d'électrons ou un groupe donneur d'électrons ; et n vaut 1-4 ; ou dans laquelle
R est le radical benzyle,
R₁ est le radical méthyle,
R₂ est l'hydrogène,
R₃ est le radical 2-(5-méthylfuryle), 2-benzofuryle, 2-benzo[b]thiényle, 2-(5-méthylpyrrolyle), ou 2-pyridyle ; et
n vaut 1 ; ou
dans laquelle
R est le radical 2-fluorobenzyle, 3-fluorobenzyle, 4-fluorobenzyle, 2,5-difluorobenzyle, ou 2,6-difluorobenzyle,
R₁ est le radical méthyle,
R₂ est l'hydrogène,
R₃ est le radical furyle, et
n vaut 1,
et ses sels acceptables en pharmacie.

2. Composé selon la revendication 1, dans lequel n vaut 1.

3. Composé selon la revendication 1 ou 2, dans lequel R₁ est le radical méthyle.

4. Composé selon l'une quelconque des revendications 1 à 3, dans lequel R est un radical arylalkyle inférieur.

5. Composé selon l'une quelconque des revendications 1 à 4, dans lequel R est un radical benzyle qui est non substitué ou substitué par un radical fluoro.

6. Composé selon l'une quelconque des revendications 1 à 5, dans lequel le groupe extracteur d'électrons est un radical halogéno, nitro, alcanoyle inférieur, aryloyle, arylalcanoyle inférieur, carboxy, carbalcoxy, carboxamido, cyano, sulfonyle, sulfoxyde, hétérocyclique, guanidine ou ammonium quaternaire.

7. Composé selon l'une quelconque des revendications 1 à 5, dans lequel le groupe donneur d'électrons est un radical hydroxy, alcoxy inférieur, alkyle inférieur, amino, alkylamino inférieur, di(alkyl inférieur)amino, phénoxy, thiol, alkylmercapto inférieur ou disulfure.

8. Composé selon l'une quelconque des revendications 1 à 7, dans lequel l'un de R₂ et R₃ est l'hydrogène.

9. Composé selon l'une quelconque des revendications 1 à 8, dans lequel l'un de R₂ et R₃ est l'hydrogène et l'autre est Z-Y.

10. Composé selon l'une quelconque des revendications 1 à 9, dans lequel Z-Y est un radical hydrazino, alkylhydrazino inférieur, N-phénylhydrazino, N-hydroxylamino, ou O-hydroxylamino.

11. Composé selon l'une quelconque des revendications 1 à 5, qui est l'un des suivants :
(D,L)-α-acétamido-N-benzyl-2-(5-méthylfurane)acétamide,
(D,L)-α-acétamido-N-benzyl-2-benzofurane-acétamide,
(D,L)-α-acétamido-N-benzyl-2-benzo[b]thiophène-acétamide,
(D,L)-α-acétamido-N-benzyl-2-(5-méthylpyrrole)acétamide,
(D,L)-α-acétamido-N-(2-fluorobenzyl)-2-furane-acétamide,
(D,L)-α-acétamido-N-(3-fluorobenzyl)-2-furane-acétamide,
(D,L)-α-acétamido-N-(4-fluorobenzyl)-2-furane-acétamide,
(D,L)-α-acétamido-N-(2,5-difluorobenzyl)-2-furane-acétamide,
(D,L)-a-acétamido-N-(2,6-difluorobenzyl)-2-furane-acétamide, Benzylamide d'acide (D,L)-2-acétamido-4-penténoïque,
(D,L)-2-acétamido-N-benzyl-2-(N-anilino)acétamide,
(D,L)-2-acétamido-N-benzyl-2-(méthylamino)acétamide, ou
(D,L)-2-acétamido-N-benzyl-2-(éthylamino)acétamide,
ou leurs isomères D ou L.

12. Composé selon la revendication 11, dans lequel le composé est le stéréoisomère D.

13. Composition anticonvulsivante comprenant une quantité anticonvulsivante efficace d'un composé selon l'une quelconque des revendications 1 à 12 et un véhicule acceptable en pharmacie pour ce composé.

14. Composition selon la revendication 13, ayant une forme posologique unitaire contenant de 5 à 1000 mg dudit composé.

15. Procédé pour la préparation d'un composé de formule I : 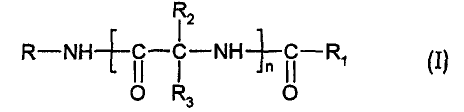 comme dans la revendication 1,
qui comprend :
a) la réaction d'une amine de formule II : 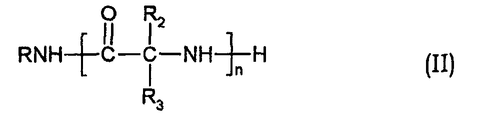 dans laquelle R, R₂ et R₃ ont la même signification que dans la formule I,
avec un dérivé d'acylation d'un composé de formule III : 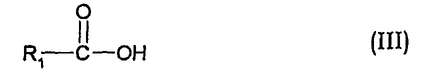 dans laquelle R₁ a la même signification que dans la revendication 1,
dans des conditions de formation d'amide ; ou
b) la réaction d'un composé de formule VI : 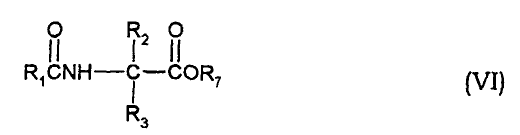 dans laquelle R₁, R₂ et R₃ ont la même signification que dans la revendication 1 et R₇ est un radical alkyle inférieur, aryle ou arylalkyle inférieur ou un de leurs dérivés d'acylation,
avec une amine de formule RNH₂ où R a la même signification que dans la revendication 1, dans des conditions de formation d'amide ; ou
c) la réaction d'un amide de formule IX : 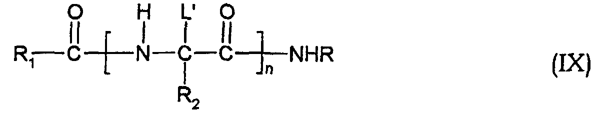
dans laquelle R, R₁, R₂ et n ont la même signification que dans la revendication 1,
avec un dérivé d'acylation de formule X :
R₃L (X)
dans laquelle R₃ a la même signification que dans la revendication 1 mais n'est pas un radical aryle, hétéroaromatique ou aromatique polynucléaire, et L et L' sont indépendamment un bon groupe partant, tel qu'un halogénure, les tosylates, les mésoylates, les brosylates et benzyloxy, dans des conditions de substitution ;
et éventuellement l'élimination de groupes protecteurs quand ils sont présents ; et éventuellement l'introduction de substituants dans les composés par des réactions de substitution ou de conversion ; et éventuellement la séparation des diastéréomères, ou éventuellement la formation de sels acceptables en pharmacie desdits composés.

16. Procédé selon la revendication 15, dans lequel n vaut 1.

17. Procédé selon la revendication 15 ou 16, dans lequel R₁ est le radical méthyle.

18. Procédé selon l'une quelconque des revendications 15 à 17, dans lequel R est un radical arylalkyle inférieur.

19. Procédé selon l'une quelconque des revendications 15 à 18, dans lequel R est un radical benzyle qui est non substitué ou substitué par un radical fluoro.

20. Procédé selon l'une quelconque des revendications 15 à 19, dans lequel le groupe extracteur d'électrons est un radical halogéno, nitro, alcanoyle inférieur, aryloyle, arylalcanoyle inférieur, carboxy, carbalcoxy, carboxamido, cyano, sulfonyle, sulfoxyde, hétérocyclique, guanidine ou ammonium quaternaire.

21. Procédé selon l'une quelconque des revendications 15 à 19, dans lequel le groupe donneur d'électrons est un radical hydroxy, alcoxy inférieur, alkyle inférieur, amino, alkylamino inférieur, di(alkyl inférieur)amino, phénoxy, thiol, alkylmercapto inférieur ou disulfure.

22. Procédé selon l'une quelconque des revendications 15 à 21, dans lequel l'un de R₂ et R₃ est l'hydrogène.

23. Procédé selon l'une quelconque des revendications 15 à 22, dans lequel l'un de R₂ et R₃ est l'hydrogène et l'autre est Z-Y.

24. Procédé selon l'une quelconque des revendications 15 à 22, dans lequel Z-Y est un radical hydrazino, alkylhydrazino inférieur, N-phénylhydrazino, N-hydroxylamino, ou O-hydroxylamino.

25. Procédé selon l'une quelconque des revendications 15 à 19, dans lequel le composé formé est choisi parmi les suivants :
(D,L)-α-acétamido-N-benzyl-2-(5-méthylfurane)acétamide,
(D,L)-α-acétamido-N-benzyl-2-benzofurane-acétamide,
(D,L)-α-acétamido-N-benzyl-2-benzo[b]thiophène-acétamide,
(D,L)-α-acétamido-N-benzyl-2-(5-méthylpyrrole)acétamide,
(D,L)-α-acétamido-N-(2-fluorobenzyl)-2-furane-acétamide,
(D,L)-α-acétamido-N-(3-fluorobenzyl)-2-furane-acétamide,
(D,L)-α-acétamido-N-(4-fluorobenzyl)-2-furane-acétamide,
(D,L)-α-acétamido-N-(2,5-difluorobenzyl)-2-furane-acétamide,
(D,L)-α-acétamido-N-(2,6-difluorobenzyl)-2-furane-acétamide, Benzylamide d'acide (D,L)-2-acétamido-4-penténoïque,
(D,L)-2-acétamido-N-benzyl-2-(N-anilino)acétamide,
(D,L)-2-acétamido-N-benzyl-2-(méthylamino)acétamide, ou
(D,L)-2-acétamido-N-benzyl-2-(éthylamino)acétamide,
ou leurs isomères D ou L.

26. Procédé selon la revendication 25, dans lequel le composé formé est le stéréoisomère D.

27. Procédé pour la préparation d'un énantiomère d'acétamido-N-benzyl-2-furane-acétamide, qui comprend la réaction d'acide α-acétamido-2-furane-acétique racémique ou d'un dérivé d'acylation de celui-ci avec l'un des stéréoisomères d'une amine optiquement active ayant un centre chiral dans des conditions efficaces pour former le sel diastéréomère correspondant, la séparation des sels diastéréomères pour l'obtention du sel souhaité, l'hydrolyse du sel dans des conditions efficaces pour former l'acide α-acétamido-2-furane-acétique souhaité et la réaction du produit ainsi formé avec une benzylamine dans des conditions de formation d'amide.

28. Procédé selon la revendication 27, dans lequel l'acide α-acétamido-2-furane-acétique racémique est préparé par réaction de furane avec un dérivé d'acylation d'acide acétamido-2-halogénoacétique où le groupe halogéno est bromo ou chloro.

29. Utilisation de composés selon les revendications précédentes pour la préparation d'un médicament destiné au traitement de l'épilepsie et d'autres troubles du SNC.

## Revendications (Revendications pour l'(les) Etat(s) contractant(s) suivant(s): ES, GR)

1. Procédé pour la préparation d'un composé de formule : 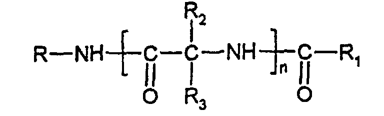 dans laquelle
R est un radical aryle, arylalkyle inférieur, hétérocyclique, hétérocycloalkyle inférieur, lequel R est non substitué ou est substitué par au moins un groupe extracteur d'électrons ou groupe donneur d'électrons ;
R₁ est l'hydrogène ou un radical alkyle inférieur, non substitué ou substitué par un groupe donneur d'électrons ou un groupe extracteur d'électrons ;
R₂ et R₃ sont indépendamment l'hydrogène ou Z-Y qui peut être non substitué ou substitué par au moins un groupe extracteur d'électrons ou un groupe donneur d'électrons, dans la mesure où R₂ et R₃ ne peuvent pas être tous deux l'hydrogène ;
Z est O, S, NR₄ ou PR₄ ;
Y est un radical arylalkyle inférieur, alcényle inférieur ou alcynyle inférieur, et Y peut être non substitué ou substitué par un groupe donneur d'électrons ou un groupe extracteur d'électrons ; ou bien
ZY, pris ensemble, est NR₄NR₅R₆, NR₄OR₅, OPR₄R₅, PR₄OR₅, SNR₄R₅, NR₄R₅, NR₄SR₅, SPR₄R₅, PR₄SR₅, NR₄PR₅R₆ ou PR₄NR₅R₆ ;
R₄, R₅ et R₆ sont indépendamment l'hydrogène ou un radical alkyle inférieur, aryle, arylalkyle inférieur, alcényle inférieur ou alcynyle inférieur, où R₄, R₅ et R₆ peuvent être non substitués ou substitués par un groupe soustracteur d'électrons ou un groupe donneur d'électrons ; et n vaut 1-4 ; ou dans laquelle
R est le radical benzyle,
R₁ est le radical méthyle,
R₂ est l'hydrogène,
R₃ est le radical 2-(5-méthylfuryle), 2-benzofuryle, 2-benzo[b]thiényle, 2-(5-méthylpyrrolyle), ou 2-pyridyle ; et
n vaut 1 ; ou
dans laquelle
R est le radical 2-fluorobenzyle, 3-fluorobenzyle, 4-fluorobenzyle, 2,5-difluorobenzyle, ou 2,6-difluorobenzyle,
R₁ est le radical méthyle,
R₂ est l'hydrogène,
R₃ est le radical furyle, et
n vaut 1,
et de ses sels acceptables en pharmacie, comprenant
a) la réaction d'une amine de formule II : 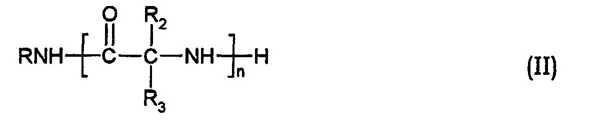 dans laquelle R, R₂ et R₃ ont la même signification que dans la formule I, avec un dérivé d'acylation d'un composé de formule III : 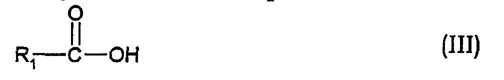 dans laquelle R₁ a la même signification que ci-dessus,
dans des conditions de formation d'amide ; ou
b) la réaction d'un composé de formule VI : 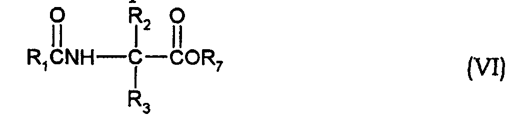 dans laquelle R₁, R₂ et R₃ ont la même signification que ci-dessus et R₇ est un radical alkyle inférieur, aryle ou arylalkyle inférieur ou un de leurs dérivés d'acylation, avec une amine de formule RNH₂ où R a la même signification que ci-dessus, dans des conditions de formation d'amide ; ou
c) la réaction d'un amide de formule IX : 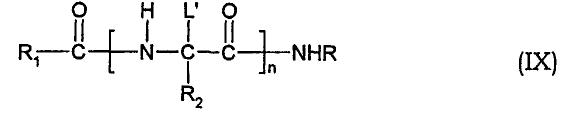
dans laquelle R, R₁, R₂ et n ont la même signification que ci-dessus, avec un dérivé d'acylation de formule X :
R₃L (X)
dans laquelle R₃ a la même signification que ci-dessus mais n'est pas un radical aryle, hétéroaromatique ou aromatique polynucléaire, et L et L' sont indépendamment un bon groupe partant, tel qu'un halogénure, les tosylates, les mésoylates, les brosylates et benzyloxy, dans des conditions de substitution ;
et éventuellement l'élimination de groupes protecteurs quand ils sont présents ; et éventuellement l'introduction de substituants dans les composés par des réactions de substitution ou de conversion ; et éventuellement la séparation des diastéréomères, ou éventuellement la formation de sels acceptables en pharmacie desdits composés.

2. Procédé selon la revendication 1, dans lequel n vaut 1.

3. Procédé selon la revendication 1 ou 2, dans lequel R₁ est le radical méthyle.

4. Procédé selon l'une quelconque des revendications 1 à 3, dans lequel R est un radical arylalkyle inférieur.

5. Procédé selon l'une quelconque des revendications 1 à 4, dans lequel R est un radical benzyle qui est non substitué ou substitué par un radical fluoro.

6. Procédé selon l'une quelconque des revendications 1 à 5, dans lequel le groupe extracteur d'électrons est un radical halogéno, nitro, alcanoyle inférieur, aryloyle, arylalcanoyle inférieur, carboxy, carbalcoxy, carboxamido, cyano, sulfonyle, sulfoxyde, hétérocyclique, guanidine ou ammonium quaternaire.

7. Procédé selon l'une quelconque des revendications 1 à 5, dans lequel le groupe donneur d'électrons est un radical hydroxy, alcoxy inférieur, alkyle inférieur, amino, alkylamino inférieur, di(alkyl inférieur)amino, phénoxy, thiol, alkylmercapto inférieur ou disulfure.

8. Procédé selon l'une quelconque des revendications 1 à 7, dans lequel l'un de R₂ et R₃ est l'hydrogène.

9. Procédé selon l'une quelconque des revendications 1 à 8, dans lequel l'un de R₂ et R₃ est l'hydrogène et l'autre est Z-Y.

10. Procédé selon l'une quelconque des revendications 1 à 5, dans lequel Z-Y est un radical hydrazino, alkylhydrazino inférieur, N-phénylhydrazino, N-hydroxylamino, ou O-hydroxylamino.

11. Procédé selon l'une quelconque des revendications 1 à 5, dans lequel le composé formé est choisi parmi les suivants :
(D,L)-α-acétamido-N-benzyl-2-(5-méthylfurane)acétamide,
(D,L)-α-acétamido-N-benzyl-2-benzofurane-acétamide,
(D,L)-α-acétamido-N-benzyl-2-benzo[b]thiophène-acétamide,
(D,L)-α-acétamido-N-benzyl-2-(5-méthylpyrrole)acétamide,
(D,L)-α-acétamido-N-(2-fluorobenzyl)-2-furane-acétamide,
(D,L)-α-acétamido-N-(3-fluorobenzyl)-2-furane-acétamide,
(D,L)-α-acétamido-N-(4-fluorobenzyl)-2-furane-acétamide,
(D,L)-a-acétamido-N-(2,5-difluorobenzyl)-2-furane-acétamide,
(D,L)-α-acétamido-N-(2,6-difluorobenzyl)-2-furane-acétamide, Benzylamide d'acide (D,L)-2-acétamido-4-penténoïque,
(D,L)-2-acétamido-N-benzyl-2-(N-anilino)acétamide,
(D,L)-2-acétamido-N-benzyl-2-(méthylamino)acétamide, ou
(D,L)-2-acétamido-N-benzyl-2-(éthylamino)acétamide,
ou leurs isomères D ou L.

12. Procédé selon la revendication 11, dans lequel le composé formé est le stéréoisomère D.

13. Procédé pour la préparation d'un énantiomère d'acétamido-N-benzyl-2-furane-acétamide, qui comprend la réaction d'acide α-acétamido-2-furane-acétique racémique ou d'un dérivé d'acylation de celui-ci avec l'un des stéréoisomères d'une amine optiquement active ayant un centre chiral dans des conditions efficaces pour former le sel diastéréomère correspondant, la séparation des sels diastéréomères pour l'obtention du sel souhaité, l'hydrolyse du sel dans des conditions efficaces pour former l'acide α-acétamido-2-furane-acétique souhaité et la réaction du produit ainsi formé avec une benzylamine dans des conditions de formation d'amide.

14. Procédé selon la revendication 13, dans lequel l'acide α-acétamido-2-furane-acétique racémique est préparé par réaction de furane avec un dérivé d'acylation d'acide acétamido-2-halogénoacétique où le groupe halogéno est bromo ou chloro.

15. Procédé pour la préparation d'un médicament destiné au traitement de l'épilepsie et d'autres troubles du SNC utilisant un composé de formule I tel que défini dans l'une quelconque des revendications 1 à 12.

16. Utilisation d'un composé de formule I tel que défini dans l'une quelconque des revendications 1 à 12,, pour la préparation d'un médicament destiné au traitement de l'épilepsie et d'autres troubles du SNC.
